# EUROPÄISCHE PATENTSCHRIFT

(11) **EP 0 374 207 B1**
(45) Veröffentlichungstag und Bekanntmachung des Hinweises auf die Patenterteilung: **21.07.1999**
(21) Anmeldenummer: 89904551.2
(22) Anmeldetag: 14.04.1989
(51) Int. Cl.: A61K 39/395, A61K 45/06

(54) **ARZNEIMITTEL ZUR BEHANDLUNG VON KREBS, AIDS UND VIRUSERKRANKUNGEN**
DRUG FOR TREATING CANCER, AIDS AND VIRAL DISEASES
MEDICAMENT POUR LE TRAITEMENT DU CANCER, DU SIDA ET DES MALADIES VIRALES

(30) Priorität: 15.04.1988 DE 3812605
(43) Veröffentlichungstag der Anmeldung: 27.06.1990
(73) Patentinhaber: LESKOVAR, Peter, D-85640 Putzbrunn (DE)
(72) Erfinder: LESKOVAR, Peter, D-85640 Putzbrunn (DE)
(74) Vertreter: Leissler-Gerstl, Gabriele
(86) Internationale Anmeldenummer: EP8900403
(87) Internationale Veröffentlichungsnummer: WO8909620

(56) Entgegenhaltungen:
- EP-A- 0 256 714

## Beschreibung

Die Erfindung betrifft ein Arzneimittel zur Behandlung von Krebs, AIDS und anderen viralen Infektionen.

### 1. Tumortherapie

Bei dem zur Zeit sehr aktuellen S. A. Rosenberg-LAK-Therapiemodell werden die peripheren Lymphozyten des Tumorpatienten mit hoch dosiertem IL-2 polyklonal aktiviert und reinjiziert. Nachteile sind die Coaktivierung von Suppressorzellen, unspezifische Aktivierung und hohe Kosten für IL-2. Bisherige tumortherapeutische Verfahren (operativer Eingriff, Chemotherapie, Radiotherapie, Kombinationstherapie) haben sich nahezu ausschließlich auf die Entfernung bzw. Abtötung der Tumorzellen konzentriert und den zweiten ebenso wichtigen Feind, die tumorprotektiven Suppressorzellen, außer Acht gelassen. Es wurde nun gefunden, daß die tumorspezifischen Suppressorzellen, die die Etablierung der Tumorzellen im Organismus durch den aktiven Schutz gegenüber der immunologischen Surveillance ermöglichen und das ungehinderte Tumorwachstum mitbestimmen. Wie aus zahlreichen Tierversuchen hervorgeht, ist eine dauerhafte Heilung, nicht nur eine temporäre Remission, nur dann möglich, wenn die Suppressorzellen vor jeglicher Immuntherapie eliminiert werden. Diese Präeliminierung der Suppressorzellen ist conditio sine qua non für alle Formen der Immuntherapie; sie gilt im Prinzip für alle Tumorarten und ist abhängig von der Individualität und genetischer Variabilität von Tumoren. Ihre generalisierte Bedeutung resultiert aus der Tatsache, daß hier nicht die individuell unterschiedlichen Tumorzellen, sondern die tumorprotektiven Suppressorzellen, die einheitliche, humanspezifische Merkmale bzw. Oberflächenstrukturen aufweisen, eliminiert bzw. inaktiviert werden; deshalb ist hier eine weitgehende Standardisierung der einzusetzenden Substanzen und Substanzgemische möglich.

Weil es auch in der Zukunft nicht denkbar ist, daß die restlichen Primärtumor- und/oder (Prämikro)metastasenzellen eliminierbar wären und somit Tumorrezidive praktisch "vorprogrammiert" sind, kann nur auf einer aktiven Bekämpfung der Resttumorzellen durch prä- oder reaktivierte tumorizide Effektorzellen aufgebaut werden; diese besitzen das feine Instrumentarium zur Erkennung und Auffindung der nach Operation, Radio- oder Chemotherapie zurückgebliebenen Resttumorzellen. Bekanntlich können aktivierte Makrophagen, aber auch NK-Zellen, transformierte Zellen erkennen und abtöten.

In Patent Abstracts of Japan, Band 9, Nr. 166 (c-290) (1989), 1985, JP-A6038328 wird ein Verfahren zur Förderung der Induktion von antineoplastischer Immunität beschrieben. Dazu wird eine Tumorzelle, die chemisch oder physikalisch so verändert wurde, daß sie sich nicht mehr vermehren kann, mit einem inaktivierten Vaccinia-Virus gekuppelt und injiziert, um eine gegen diese Tumorzelle gerichtete Immunantwort hervorzurufen. Diese Therapie kann nur spezifisch für eine Krebsart eingesetzt werden. Die Suppressorzellen werden bei dieser Therapie nicht elimiert.

Weiterhin wird in EP-A 0 256 714 vorgeschlagen, zur Behandlung von Krebs eine Kombination eines Immunotoxins und von Interleukin-2 zu verwenden. Suppressorzellen werden durch diese Behandlung nicht erfaßt.

Es war nun Aufgabe der Erfindung, ein Mittel bereitzustellen, mit dem tumorprotektive Suppressorzellen eliminiert werden können und anschließend Effektorzellen, die die Tumorzellen angreifen, aktiviert werden.

Diese Aufgabe wird gelöst mit dem in Anspruch 1 definierten Arzneimittel.

Bei der erfindungsgemäßen, von der Tumorart unabhängigen Immuntherapie werden (nach der operativen Entfernung der Tumorhauptmasse) in der Phase I (Immun-Derepression) tumorprotektive Suppressorzellen des Patienten (z.B. mit monoklonalen Antikörpern) eliminiert bzw. coeliminiert.

In der anschließenden Phase II (Prä- bzw. Reaktivierung der Patienten-Immunabwehr) werden die Patienten-Effektorzellen, insbesondere bei tumoriziden Effektorzellen, entweder ex vivo präaktiviert und dem Patienten reinjiziert oder aber direkt in vivo reaktiviert, was nach der vorausgegangenen Suppressorzell-Präeliminierung (Phase I) möglich wird. Unter Effektorzellen werden hier Makrophagen, NK-Zellen und K/ADCC-Zellen verstanden.

Wenn im folgenden von Antikörpern, auch als AK bezeichnet, die Rede ist, so sind darunter hier, wenn nicht ausdrücklich anders vermerkt, monoklonale und polyklonale Antikörper sowie deren Fragmente, insbesondere Fab und (Fab')₂-Untereinheiten zu verstehen.

Die Eliminierung/Coeliminierung von Suppressorzellen kann spezifisch oder generell (tumor-unspezifisch) unspezifisch erfolgen. Geeignet sind für Komponente A: Antikörper, die spezifisch für Suppressor-Zellen sind, z.B. Anti-CD8/Leu2/T8(T5)-Mab; spezifisch für Suppressor-Inducer-Zellen sind, z.B. Kombination aus (subcytolytisch zu dosierenden) Antikörper, gerichtet gegen die auf der Zelloberfläche dieser Suppressor-Präkursor-Zellen befindlichen Antigene Leu3(CD4/T4), Leu8 und Leu18(CD45R); spezifisch für Suppressor-Transducer-Zellen sind, z.B. Anti-CD1/Leu6/T6-AK, Anti-CD38/ Leu17/T10-AK; spezifisch für eine Subpopulation von radiosensitiven Suppressor(Inducer)-Zellen sind, z.B. Anti-CD4/Leu3/T4-AK; spezifisch für immunkompetente (pan)-T-Zellen sind, z.B. Anti-CD3/Leu4/T3, Anti-CD5/Leu1/T1-AK, Anti-CD2/Leu5b/T11-AK, zum Teil Anti-CD7/Leu9-AK sowie Antikörper gegen die Oberflächenantigene Leu7 und Leu8; spezifisch für (pan)-Leukozyten sind, z.B. Anti-CD45/HLe-1-Mab sowie Antikörper gegen das Oberflächenantigen Leu8; polyklonale/polyvalente Antikörper bzw. Globuline (1) gegen T-Zellen und Thymozyten und (2) gegen Lymphozyten, nach Art von ATS/ATG bzw. ALS/ALG.

Es können auch Antikörper gegen aktivierte T-Zellen mit sehr schneller Wirkung eingesetzt werden. Solche Antikörper sind: Anti-Transferrin-Rezeptor-AK/Anti-T9-AK, ferner Anti-CD25/IL-2-Rezeptor/Tac-AK sowie Anti-CD38/Leu-17/T10-AK.

Die genannten Antikörper können auch kombiniert werden, bevorzugt sind z.B. die Kombination von Anti-CD8/Leu2/T8-AK mit Anti-CD4/Leu3/T4-AK, Anti-CD3/Leu4/T3-AK, Anti-CD5/Leu1/T1-AK und/oder Anti-CD2/Leu5b/T11-AK. Eine weitere bevorzugte Kombination ist Anti-CD8/Leu2/T8-AK mit Anti-CD45R/Leu18 und/oder Anti-CD4/Leu3/T4 sowie Anti-CD11b-AK.

Vorteilhaft ist es, für Komponente A Heterokonjugate zu verwenden, bei denen ein Teil spezifisch oder unspezifisch mit Suppressorzellen bindet und der andere Teil entweder toxisch für Suppressorzellen ist oder die Vernichtung dieser Zellen bewirken kann. Beispiele hierfür sind:
(a) RES-abhängig: Heterokonjugate aus AK der IgG-Subklasse (bindefähig mit Suppressorzellen) und Staphylococcus aureus-Protein A; Heterokonjugate aus AK und Anti-Spezies (Maus)-Ig (mit bzw. ohne Complement); Heterokonjugate aus AK und humanem, präaggregiertem IgG (mit bzw. ohne Complement); Heterokonjugate aus AK und Complement bzw. Complement-Untereinheiten; Heterokonjugate aus AK und xenogenen Proteinen.
(b) RES-unabhängig: Konjugate von gegen Suppressorzellen gerichteten Antikörpern oder deren Fragmenten mit Zelltoxinen ("Immuntoxine"), wie Ricin bzw. Ricinalpha-Kette, Abrin, Diphterie-Toxin/Toxoid, Purothionin, Modeccine, Pseudomonas-Exotoxin, Gelonin oder Pokeweed-Toxin; mit Radionukliden, wie ^{131/125}I, ¹¹¹In, ⁹⁹Tc oder ⁹⁰Yt oder mit Cytostatika, wie Doxorubicin, Adriamycin, Daunomycin, Mitomycin C, Methotrexate, Vinkaalkaloide oder Anthrazykline.

Die Xenogenisierung der Antikörper zur beschleunigten RES-Eliminierung kann mit Paratop/Idiotyp-erhaltender Behandlung mit Formaldehyd oder Glutar(di)aldehyd erzielt werden; dies gilt für alle Antikörper und deren Konjugate/Heterokonjugate.

Um eine Neutralisierung heterologer, z.B. muriner Antikörper bei wiederholter Behandlung zu verhindern, können verschiedene Maßnahmen ergriffen werden. Es kann die Spezies des Antikörpers bei gleicher Spezifität variiert werden. Besser geeignet ist die Verwendung von Anti-CD4/Leu3/T4-Mab und/oder Anti-B-Mabs (z.B. Anti-CD21) und/oder Anti-HLA-DR und/oder Anti-CD25- und/oder Anti-T9- Mab, welche die Bildung von neutralisierenden Antikörpern unterbinden. Anstelle von Anti-CD25-Mab kann ein Konjugat aus IL-2 und einem Cytotoxin verwendet werden. Weiterhin können die Antikörper, bevorzugt deren Fab/F(ab')₂-Untereinheiten mit humanen Serumproteinen, bevorzugt IgG, besonders bevorzugt mit IgG-Aggregaten bzw. mit Formaldehyd oder Glutaraldehyd vorbehandeltem IgG mit starken Tolerogenen wie D-GL, isologe IgG, PEG, PVP, gekoppelt sein.

Eine andere Variante besteht darin, Konjugate aus xenogenen Proteinen, insbesondere Immunglobuline mit Cytotoxinen, bevorzugt zusammen mit Konjugaten aus den gleichen xenogenen Proteinen und Tolergenen (D-GL) der Komponente A zuzusetzen. Ebenso besteht die Möglichkeit der Toleranzinduktion nach der Chiller/Weigle-Methode. Dazu werden toleranzinduzierende, hoch gereinigte, aggregatfreie, heterologe Proteine, bevorzugt speziell gereinigte, von Teilaggregaten befreite Antikörper vor der Behandlung mit dem nicht vorgereinigten Antikörper dem Patienten injiziert. Eine andere Möglichkeit besteht darin, heterologe Proteine, gegen die Toleranz induziert werden soll, oral zu verabreichen. Die so induzierte Immuntoleranz folgt dem Sulzberger/Chase-Prinzip.

In einer bevorzugten Ausführungsform werden für Komponente A die oben aufgeführten Antikörper und (Hetero)konjugate mit Suppressorzell-schwächenden Substanzen, wie Indomethazin, Cimetidin, Theobromin, Caffein, Theophyllin, Cycloheximid, Aminophyllin, Desoxyguanosin, Isoproterenol und Histamin-Antimetaboliten kombiniert.

Bevorzugt werden auch für Komponente A Kombinationen mit Suppressor-spezifischen Lektinen wie Concanavalin A (ConA) und/oder PNA (peanut agglutinin) sowie mit einigen sub-dosierten Cytostatika, in erster Linie Alkylantien (Cyclophosphamid) eingesetzt.

Um auch die gegebenenfalls durch die angewendeten Substanzen von Komponente A nicht erfaßten Vorläufer-Suppressorzellen zu inaktivieren, können zusätzlich noch mit diesen Zellen bindefähige Antikörper eingesetzt werden, z.B. mit CD1/Leu6/T6 oder CD38/Leu17/T10 bindefähige Antikörper. Ebenso sind hierfür auch gegen das auf Vorläuferzellen exprimierte TdT (terminal Deoxynukleotidyl Transferase)-Antigen geeignet.

Zur tumorunspezifischen Coeliminierung von aktiven/aktivierten Suppressorzellen können, da die aktiven/aktivierten Suppressor-T-Zellen ähnlich wie andere aktivierte T-Zell-Subpopulationen CD25/IL-2(Tac)-Rezeptoren, Leu17/T10-Oberflächenantigen und/oder T9/Transferrin-Rezeptor exprimieren die entsprechenden damit bindefähigen Antikörper ggf. in Form von Konjugaten aus Liganden (IL-2; Transferin) und Cytotoxinen verwendet werden. Geeeignet sind dazu beispielsweise Anti-CD25-AK, Anti-Leu17-AK, Anti-T9-AK sowie entsprechende Immuntoxine, IL-2-Konjugate mit Cytotoxinen und/oder Transferrin-Konjugate mit Cytotoxinen.

Die Antikörper und die daraus gebildeten Immuntoxine können mit den Ligand: Cytotoxin-Konjugaten kombiniert werden, um die Wirkung weiterhin zu verstärken.

In einer weiteren bevorzugten Ausführungsform der Erfindung werden zur selektiven Eliminierung der Suppressorzellen mehrere mit Suppressorzellen und deren Vorläuferzellen reagierende Antikörper in sublytischer Dosis verwendet, wobei dann nur die Zielzellen, d.h. Suppressorzellen, RES-eliminiert werden, die durch Anlagerung mehrerer Antikörper an der Zelloberfläche die kritische Antikörper-Grenzdichte überschreiten. In einer Variante hierzu werden die Oberflächenstrukturen mit Antikörpern überbrückt, wobei dann an so "markierte" Zielzellen cytotoxische Substanzen gekoppelt werden können.

Für Komponente A können beispielsweise Kombinationen von AK in sub-lytischer Dosis von Anti-CD8/Leu2/T8(T5)-AK und Anti-CD11b/Leu15/CR₃-AK oder Anti-CD4/Leu3/T4-Mab und Anti-Leu8-Mab und Anti-CD45R/Leu18/-Mab in freier Form oder konjugiert mit Cytotoxinen, Cytostatika oder Radionukliden verwendet werden.

Alternativ hierzu können die Suppressorzellen und ihre Präkursoren durch Fab- bzw. F(ab')₂-Untereinheiten der erwähnten Antikörper-Gemische und durch die anschließende Quervernetzung mit Anti-Fab(F(ab')₂-Antikörpern "markiert" werden; die Konjugierung des brückenbildenden Anti-Fab(F(ab')₂-Ig mit Cytotoxinen, Cytostatike oder Radioisotopen unterstützt oder ersetzt die RES-Eliminierung.

Nach einer anderen Variante können die Suppressorzellen bzw. deren Präkursoren durch ein entsprechendes AK-Gemisch und die anschließende Brückenbildung ("cross-linking") mittels Anti-Species(Maus)-AK bzw. AK-Untereinheiten "markiert" und zur Beschleunigung der Zielzell-Lyse noch mit obengenannten cytotoxischen Komponenten konjugiert werden.

Eine weitere Variante ist die Beladung eines Suppressorzell-spezifischen Oberflächenantigens mit der Fab(F(ab')₂-Untereinheit des korrespondierenden AK und die Quervernetzung mittels eines Konjugats aus dem AK gegen das 2. (und 3.) Oberflächenantigen mit dem Anti-Fab(F(ab')₂-Ig.

Das gleiche Ziel kann auch durch die Kombination von Biotin-konjugierten AK, mit Avidin oder Anti-Biotin-Globulin konjugierte AK erreicht werden, wobei die AKs jeweils gegen verschiedene Oberflächenantigene der Suppressorzelle gerichtet sind. So ist z.B. eine Kombination von Anti-CD3- oder Anti-TCR-1/WT31-, z.T. auch Anti-CD2-AK mit Avidin- oder mit Anti-Biotin-Ig-konjugierten Antikörpern, die für die interessierende T-Zell-Subklasse spezifisch sind, vorteilhaft. Bei einer anderen Variante wird der Biotin- konjugierte mitogene AK durch deren Fab- bzw. F(ab')₂-Untereinheiten und der Avidin- oder Anti-Biotin-Ig-konjugierte AK durch die entsprechenden Konjugate mit den Mab-Untereinheiten ersetzt.

Alternativ zur letzten Variante können die AK gegen mehrere Suppressor-zellspezifische Antigene gerichtet sein, die alle mit Biotin konjugiert sind, so daß sie miteinander nicht "kombinieren", mit Anti-Biotin-Antikörper oder Bis/Tris-Avidin-Konjugaten quervernetzt werden.

Die nach obigen Varianten "markierten" Suppressorzellen können direkt RES eliminiert, oder - nach Konjugation mit Zelltoxinen - RES-unabhängig inaktiviert werden.

Durch Kopplung von Cytotoxinen an Antigene bzw. an entsprechende Anti-Idiotypen (bzw. deren Fab/F(ab')₂-Untereinheiten) können selektiv die tumorspezifischen, Idiotyp-tragenden T- und B-Zellen eliminiert werden. Obwohl neben den tumor-spezifischen Suppressor-T-Zellen auch die evtl. existierenden, aber ineffektiven, da blockierten cytotoxischen T-Zellen, sowie die tumorspezifischen B-Zellen, deren Rolle problematisch bzw. negativ ist, miteliminiert werden, ist dies belanglos, da diese Zellen bald aus den Präkursorzellen nachgebildet werden. Wesentlich ist die Durchbrechung der tumorspezifischen Toleranz durch Eliminierung der Suppressorzellen.

Diese tumor-spezifische Suppressoreliminierung ist nur bei bekannten Tumorantigenen möglich.

Um eine Nachrekrutierung der tumor-spezifischen Suppressorzellen einzuschließen, wird die Kombination mit Konjugaten aus Antigen bzw. Anti-Idiotyp (Fab/F(ab')₂-Untereinheit) plus Tolerogen (z.B. D-GL) empfohlen.

Ein alternativer Weg sieht vor, durch die Prägenerierung von tumor-spezifischen Anti-Idiotyp-Subklonen der T- und/oder B-Zell-Reihe die Neuentstehung der Idiotyptragenden Suppressorzellen nach deren Eliminierung zu unterbinden. Die Basis für diesen alternativen oder ergänzenden Weg ist die immunologische Netzwerktheorie nach Jerne und Wigzell.

Für diese Ausführungsform geeignet sind Konjugate aus Tumorantigenen bzw. aus den Anti- Idiotyp-Antikörpern oder deren Untereinheiten mit Cytotoxinen und/oder starken Tolerogenen (z.B. D-GL) ggf. zusammen mit Anti-Idiotypen bzw. deren Fab/F(ab')₂-Untereinheiten eventuell kombiniert mit Anti-Tumor-Antikörpern als alleinige, nicht-suppressive Behandlung. In einer bevorzugten Ausführungsform werden durch Präaktivierung bzw. klonale Präexpansion bei der Eliminierung neben den aktiven Suppressorzellen auch deren Präkursoren (Inducer- und/oder Transducer-Suppressorzellen) weitgehend miterfaßt. Durch mutogene Lektine (ConA, PNA) und/oder subdosierte mitogene Antikörper wie Anti-CD3-, Anti-Ti- oder Anti-CD2-Antikörper und/oder IL-2 bzw. IL-3 werden die klonal präexpandierten Suppressorzellen des Patienten zur weiteren Proliferation angeregt und anschließend mit Alkylantien wie Cyclophosphamid; Anti-CD8-Antikörpern und/oder Anti-CD25-(Anti- T9-, Anti-HLA-DR-)-AK bzw. entsprechenden Immuntoxinen und/oder Konjugaten aus IL-2 oder Transferrin mit Cytotoxinen eliminiert.

Die zweite wesentliche Komponente B des erfindungsgemäßen Arzneimittels bewirkt die Aktivierung der Effektorzelle. Die Aktivierung kann entweder in vitro an den von Patienten entnommen Blutproben oder in vivo erfolgen.

Für die in vitro bzw. ex vivo Aktivierung der Patienten-Effektorzellen ebenso wie für die in vivo-Aktivierung, können alle Substanzen verwendet werden, die T-Zellen zur Proliferation anregen. Geeignet sind hierzu insbesondere Lymphokine und Monokine. Bevorzugt werden IL-2, IL-1, M-CSF, GM-CSF, IL-3, IL-2 plus M-CSF bzw. GM-CSF bzw. IL-3 allein oder in Kombination zur in vitro Präaktivierung von Effektorzellen der Lymphozyten-Zellkultur des Patienten zugesetzt.

Um das Wachstum der unerwünschten Suppressorzellen in diesen Zellkulturen zu verhindern, kann zusätzlich auch hier Anti-CD8/Leu2/T8-AK, allein oder kombiniert mit Anti-CD4/Leu3/T4-AK und/oder Anti-CD45R/Leu18-AK, Anti-CD45R/leu18-AK allein oder kombiniert mit Anti-CD8/Leu2/T8-AK verwendet werden.

Weitere Kombinations-Präparate enthalten die erwähnten Antikörper (mit oder ohne die einzelnen aufgeführten Cytokine bzw. hämatologische Wachstumshormone) und sub-dosierte mitogene Antikörper wie (Anti-CD3/Leu4/T3-AK, Anti-TCR-1/WT31(Ti)-AK, Anti- CD2/Leu5/T11-AK und/oder subdosierte mitogene Lektine (PHA). Ein weiterer Bestandteil solcher AK-Kombinationspräparate können Immunmodulatoren (BRM, "biological response modifiers"), wie MDP, MTP-PE und/oder inaktivierte Bakterien, wie C. parvum oder BCG sowie Bakterien-Lysate sein.

Geeignet für Komponente B sind auch Interferon sowie Anti-PGE2 (Prostaglandin E2) und/oder Anti-Corticosteroid-Antikörper.

In einer bevorzugten Ausführungsform enthält Komponente B Heterokonjugate in Kombination mit IL-2 und/oder IL-1.

Für die Heterokonjugate gibt es verschiedene Varianten. So können z.B. zur unspezifischen/polyklonalen oder tumor-spezifischen/monoklonalen Stimulierung der Immunozyten Heterokonjugate, die T4- bzw. T8-Zellen durch Überbrückung ("cross linking") immunrelevanter Membranstrukturen aktivieren, verwendet werden. Diese Heterokonjugate verdanken ihre Wirkung dem Grundprinzip, daß eine Quervernetzung bzw. Überbrückung ("cross-linking") von immunrelevanten Oberflächenstrukturen immunkompetenter Zellen, vor allem T4- und T8-Zellen aktiviert wird. Im Prinzip handelt es sich um eine Simulierung der Vorgänge, die physiologischerweise zur Zellaktivierung führen, z.B. bei T4-Zellen durch Kurzschließung ("cross-linking") der CD3/Ti- mit den CD4/-Molekülen (via HMC classII-Strukturen auf Makrophagen) und bei T8-Zellen durch cross-linking von CD3/Ti mit CD8(via MHC class I). Durch gezielten immunregulatorischen Eingriff mittels dieser Heterokonjugate kann der bei Patienten mit Krebs, AIDS oder anderen (retro)viralen Infektionen gestörte erste Schritt, die Antigen-Präsentierung durch Makrophagen und andere APC, der zur Immuntoleranz führt, umgangen werden.

Diese Quervernetzung der immunrelevanten Oberflächenstrukturen ("cross-linking") durch Heterokonjugate betrifft folgende Membranmoleküle bei T4- bzw. T8-Zellen:

Das "cross-linking" von CD4 mit CD3/Ti zur Aktivierung von T4-Zellen; von CD8 mit CD3/Ti zur Aktivierung von T8-Zellen; von Fc-Rezeptor mit IgM mit CD3/Ti(Aktivierung von T4("T_{M}")-Zellen und von Fc-Rezeptor für IgG mit CD3/Ti(Aktivierung von T8("T_{G}")-Zellen.

Zur unspezifischen/polyklonalen Aktivierung von T4-Zellen sind Heterokonjugate aus Anti-CD3/Ti-AK, bzw. aus Anti-CD3-AK oder Anti-Ti-AK mit dem HLA-DR-Molekül oder mit Anti-CD4-AK sowie Heterokonjugate aus mitogenen Lektinen wie PHA mit dem HLA-DR-Molekül oder Anti-CD4-AK geeignet. Anstelle der Antikörper können die entsprechenden Fab- bzw. F(ab')₂-Untereinheiten und anstelle von Lektin dessen Untereinheiten (Spaltprodukte) im Konjugat eingesetzt werden.

Zur unspezifischen Aktivierung von T8-Zellen können Heterokonjugate von Anti-CD8-AK, bzw. dessen Untereinheiten mit HLA-A,B,C-Molekül verwendet werden.

Die tumorspezifische/monoklonale Aktivierung von T4-Zellen kann mit Heterokonjugaten von Tumorantigen mit HLA-DR oder Anti-CD4-Antikörper sowie Heterokonjugaten aus Anti-Idiotyp-Antikörpern des Tumorantigens und aus dem HLA-DR-Molekül oder Heterokonjugaten aus dem Anti-Idiotyp des Tumorantigens und aus Anti-CD4-AK erfolgen.

Anti-Idiotyp-Antikörper des Tumorantigens werden durch Immunisierung des 1. Tieres (z.B. Maus) mit Tumorantigen und durch die Immunisierung des 2. Tieres mit der Fab/F(ab')₂-Untereinheit des Tumorantigen-spezifischen Immunglobulins und Isolierung des Anti-Idiotyp-Globulins gewonnen.

Für die tumorspezifische Aktivierung von T8-Zellen sind Heterokonjugate von Anti-CD8-AK mit HLA-A,B,C oder mitogene Lektine bzw. deren Untereinheiten (Spaltprodukte) als Konjugatbestandteil geeignet.

Für Komponente B sind auch Heterokonjugate geeignet, die T4- bzw. T8-Zellen durch Antikörper-gesteuerte Interaktion mit Makrophagen (und anderen APC) aktivieren.

Diese Art der unspezifischen/polyklonalen bzw. tumorspezifischen/monoklonalen T-Zellaktivierung basiert auf einer immunregulatorisch gesteuerten T-Zell-Interaktion mit dem Makrophagen bzw. APC. Auch hier handelt es sich im Prinzip und eine Simulierung physiologischer T:APC-Interaktion durch gezielte Zellannäherung mittels zellspezifischer Antikörper. Diese T:APC-Interaktion, mit der Folge der T-Zellaktivierung, wird durch Heterokonjugate bzw. hybride Antikörper von Antikörpern gegen Makrophagen (APC)-Oberflächen-Antigene und gegen den CD3/Ti-Antigen-Rezeptor auf T4- und T8-Zellen herbeigeführt.

Für die unspezifische/polyklonale Aktivierung von T4- und T8-Zellen können Heterokonjugate von Anti-CD11c/Leu-M5-AK, Anti-CD14/Leu-M3-AK oder Anti-CD15/Leu-M1-AK mit Anti-CD5/Leu1/T1-AK oder Anti-CD2/Leu5b/T11-AK, Heterokonjugate von Anti-CD16/Leu11a(b,c)-AK mit Anti-CD5/Leu1/T1-AK oder Anti-CD2/Leu5b/T11-AK oder Heterokonjugate von Anti-CD11c/Leu-M5-AK, Anti-CD14/Leu-M3-AK, Anti-CD15/Leu-M1-AK oder Anti-CD16/Leu11a(b,c)-AK mit Anti-CD3/Ti-AK verwendet werden.

Statt Anti-CD3 mit Ti-AK können auch mitogene Lektine wie PHA bzw. deren Untereinheiten (Spaltprodukte) eingesetzt werden.

Zur tumorspezifischen/monoklonalen Aktivierung von T4- und T8-Zellen können Heterokonjugate von Tumorantigen oder entsprechenden Anti-Idiotyp-Antikörpern mit Anti-CD11c/Leu-M5-AK, Anti-CD14/Leu-M3-AK, Anti-CD15/Leu-M1-AK oder Anti-CD16/Leu11a(b,c)-AK verwendet werden. Auch hier kann der Anti-CD3/Ti-AK durch mitogene Lektine bzw. ihre Spaltprodukte im Konjugat ersetzt werden.

Die genannten Heterokonjugate, die auch als hybride Antikörper bezeichnet werden, können nach an sich bekannten Methoden durch chemische Kopplungstechniken, wie sie z.B. in der Affinitätschromatographie zur Proteinkopplung (Immobilisierung) verwendet werden, oder aber durch Hybridoma-Bildung 2. Generation, d.h. durch Fusionierung von Hybridomazellen, die Mab1 produzieren, mit Hybridomazellen, die Mab2 als 2. Bestandteil des Heterokonjugats produzieren, hergestellt werden. Bevorzugtes Kopplungsreagenz ist die heterobifunktionelle Verbindung SPDP(N-Succinimidyl-3-(2-pyridyldithio)-propionat).

Eine Immunstimulation kann durch Überbrückung des CD3- oder des Ti- oder des CD2-Moleküls mit dem Fc-Rezeptor für IgM (Aktivierung von T4-Zellen) bzw. für IgG (Aktivierung von T8-Zellen) bewirkt werden. Für eine unspezifische Aktivierung der T4-Zellen können daher für Komponente B auch mitogene Antikörper des IgM-Isotyps direkt oder als Heterokonjugate, bestehend aus diesen mitogenen Antikörpern und dagegen gerichteten (z.B. Kaninchen-Anti-Maus-Ig)-Antikörpern des IgM-Isotyps, verwendet werden. Alternativ können die Heterokonjugate auch Fab/F(ab')₂-Untereinheiten des mitogenen Antikörpers und den Fc-Teil des IgM-Isotyps (Fc µ) enthalten. Mitogene Antikörper sind Anti- CD3-, Anti-CD2-, Anti-Ti- und evtl. Anti-CD5-Antikörper. Entsprechend kann eine unspezifische T8(T_{G})-Aktivierung durch Ersatz des IgM-Isotyps durch den IgG-Isotyp erreicht werden.

Für die spezifische Aktivierung der T4- bzw. T8-Zellen können im Prinzip die gleichen Heterokonjugate wie bei der unspezifischen T-Zell-Stimulierung verwendet werden, wobei die mitogenen Antikörper durch das Antigen bzw. den entsprechenden Anti-Idiotyp-Antikörper ersetzt werden. Es ist vorteilhaft, anstelle des Antigens seine Fragmente, d.h. enzymatische Abbauprodukte, und anstelle von Anti-Idiotyp-Antikörpern die 6-15-Aminosäuren-haltigen peptidischen Fragmente zu nehmen, da hierdurch die Aktivierung erleichtert und das Spektrum der für einzelne Epitope des Antigens spezifischen Klone erweitert wird.

T-Zellen können auch durch eine gezielte, Makrophagen-unabhängige Kurzschließung des Rezeptors für Signal 1 mit dem Rezeptor für Signal 2 auf der Oberfläche der T-Zelle aktiviert werden.

Eine weitgehend APC-unabhängige Aktivierung der T4-Zellen kann durch spezielle Heterokonjugate, bestehend aus Signal 1 (Antigen bzw. Mitogen) und aus Signal 2 (IL-1 oder Tp44 bzw. Tp67) erzielt werden. Entsprechend kann eine T8-Aktivierung durch Heterokonjugate, bestehend aus Signal 1 (Antigen bzw. Mitogen) und aus Signal 2 (IL-2) erreicht werden. Tp44 bzw. Tp67 können durch entsprechende Fab/F(ab')₂-Untereinheiten ersetzt werden.

Für die unspezifische (polyklonale) Aktivierung von T4-Zellen können daher als Komponente B Heterokonjugate aus einem Mitogen (mitogenem Antikörper bzw. seiner Fab/F(ab')₂-Untereinheit oder Lektin bzw. seiner Untereinheit) und IL-1 oder Tp44 bzw. Tp67, eingesetzt werden. Die beiden Antikörper Tp44 und Tp67 können IL-1 als Signal 2 ersetzen. Sie wirken synergistisch auf eine T-Zell-Aktivierung durch mitogene Antikörper (Anti-CD3- bzw. Anti-Ti-Mab).

Die pezifische (monoklonale) Aktivierung von T4-Zellen wird in antigen-spezifischer Weise mit Heterokonjugaten, bestehend aus dem Antigen bzw. Anti-Idiotyp-Antikörper und IL-1 oder Tp44 bzw. Tp67 bewirkt.

Auch für diese Ausführungsform ist es bevorzugt, eine hydrolytische Prädegradierung des Antigens bzw. des entsprechenden Anti-Idiotyp-Antikörpers vorzunehmen, um Heterokonjugate mit höherer Effizienz und breiterer Klonaktivierung zu erhalten. Auch in diesen Konjugaten können Tp44 und Tp67 durch entsprechende Fab/F(ab')₂-Untereinheiten ersetzt werden. Weiterhin ist es möglich, T4-Zellen durch eine Kombination von IgM mit Anti-IgM-Antikörpern zu aktivieren.

Für die unspezifische (polyklonale) Aktivierung von T8-Zellen kann Komponente B Heterokonjugate enthalten, die aus einem Mitogen (mitogenem Antikörper bzw. seiner Fab/F(ab')₂-Untereinheit oder Lektin bzw. seiner Untereinheit) und IL-2 bestehen. Vorteilhaft werden diese Heterokonjugate mit solchen, bei denen IL-2 durch IL-1 bzw. Tp44 (Tp67) ersetzt ist, oder mit freiem IL-1 kombiniert, da die T8-Zellen für die Differenzierung und Proliferation auch IL-1 benötigen.

Eine spezifische (monoklonale) Aktivierung von T8-Zellen kann mit Heterokonjugaten, bestehend aus Antigen bzw. Anti-Idiotyp-Antikörpern und IL-2 erfolgen. Antigen- bzw. Anti-Idiotyp-Antikörper, möglichst angereichert durch eine Idiotyp-Chromatographie, d.h. Passage durch eine, mit dem spezifischen Antikörper gegen das betreffende Antigen (Tumorantigen) beladene Säule, sind als Bestandteile der Heterokonjugate gegenüber en "nicht-prozessierten" Antigenen bevorzugt. Auch hier sind Kombinationen mit Heterokonjugaten, bei denen IL-2 durch IL-1 ersetzt ist oder mit freiem IL-1 besonders bevorzugt.

Eine weitere Variante der Erfindung macht sich die Stimulierung der Effektorzellen durch Kopplung von einigen, diese Effektorzellen aktivierende Enzyme an Antikörper mit Spezifität für diese Effektorzellen zunutze. Es wurde gezeigt, daß Enzyme wie Proteasen, Lipasen, Mucopolysaccharidasen und Lysozym in kurzer Zeit Effektorzellen wie Makrophagen und NK-Zellen, stark aktivieren können. Nachdem eingehende eigene Untersuchungen zeigten, daß Eine enzymatische (proteolytische) Vorbehandlung der Effektorzellen ergibt schon innerhalb von 5 bis 10 Minuten (nach einer 30- bis 60-minütigen Regenerationsphase) eine Aktivierung von bis zu 700 % bei Makrophagen und eine Aktivierung von bis zu 1300 % bei NK-Zellen. Geeignete Enzyme sind z.B. Proteasen wie Trypsin, Chymotrypsin, Papain, Bromelin sowie andere tierische und pflanzliche Proteasen, incl. medizinisch (für andere Indikationen) eingesetzte Urokinasen, Streptokinasen, t-PA ("tissue plasmine activator"), Lipasen (A1, A2, B, C); Mucopolysaccharidasen und sonstige Enzyme, die die Oberfläche der Effektorzellen verändern und somit diese Effektorzellen aktivieren.

Da einige der getesteten Enzyme auch von aktivierten Makrophagen als Exoenzyme ausgeschieden werden, wird angenommen, daß diese Hydrolasen nicht nur dem Abbau der Zielzelle, sondern auch der Autoaktivierung des Makrophagen sowie der Coaktivierung der kooperierenden Effektorzelle (z.B. NK-Zelle) dienen.

Um die Effektorzelle gezielter zu aktivieren, werden als Komponente B bevorzugt Heterokonjugate von Enzymen (Proteasen, Lipasen, Mucopolysaccharidasen, insbesondere Lysozym und Neuraminidase/Sialidase) mit Effektorzell-spezifischen Antikörpern verwendet. Diese Antikörper können Spezifität für Makrophagen (z.B. CD11c/CD14/CD15), NK-Zellen (CD16, Leu19) oder CTLs (CD3/CD2/CD5) aufweisen.

Um eine Coaktivierung etwa vorhandener Suppressor-Monozyten zu verhindern, werden die Makrophagen/Monozytenspezifischen Antikörper (z.B. CD11c/CD14) bevorzugt mit Prostaglandin-Inhibitoren (z.B. Indomethacin, Aspirin) konjugiert.

Um die vorzeitige Neutralisierung der von aktivierten Makrophagen sezernierten Proteasen durch ebenso von Makrophagen ausgeschiedenes alpha2-Makroglobulin und alpha1-Proteinase-Inhibitor zu verhindern, können Heterokonjugate aus den gleichen Anti-Makrophagen/Monozyten-Antikörpern mit Anti-alpha2-Makroglobulin, Antialpha1-Proteinase-Inhibitor, Anti-Trypsin und/oder Anti-Chymotrypsin verwendet werden.

Besonders bevorzugt wird zur direkten Makrophagen-Aktivierung ein Konjugat aus Anti-Makrophagen/Monozyten-Antikörpern mit Endotoxin oder gamma-Interferon eingesetzt.

Da die antimikrobielle und tumorizide Wirkung der Makrophagen auf der Oxydation durch Peroxid (O₂⁻), Wasserstoff-Peroxid (H₂O₂), Hydroxyl-Radikal (OH^{·}) und atomarem O₂ (¹O₂) basiert (Klebanoff), werden in einer weiteren Variante die Anti-Makrophagen/Monozyten-Antikörper mit (Myelo)peroxidase und/oder Katalase konjugiert. Der Effekt kann durch Kopplung von Anti-Glutathion-Peroxidase und/oder Anti-Glutathion-Reductase potenziert werden. Günstig ist der Zusatz von Haliden wie Cl⁻oder J⁻.

Das Konjugat der gleichen Antikörper mit Dibutyryl-cGMP aktiviert die Zielzelle durch Ca²⁺-Influx; es kann mit dem Konjugat aus Mab plus Ionophor (z.B. Ionophor A 23187), welches das Ca²⁺ aus dem intrazellulären Depot mobilisiert, kombiniert werden.

Konjugate der Anti-Makrophagen/Monozyten-Antikörper mit Theophyllin, Theobromin, Coffein, Catecholaminen (incl. Epinephrin, Norepinephrin und Isoproterenol) und anderen CAMP-(Adenyl-cyclase-)Stimulatoren können für eine selektive Effektorzell-Aktivierung in Komponente B verwendet werden.

Makrophagen können auch durch Konjugate aus Anti-Makrophagen/Monozyten-Antikörpern und Complement-Untereinheiten wie C1, C2, C3, C4, C5, C3bi, C5a aktiviert werden. In Analogie zu Mastzellen, die durch IgE und Anti-IgE oder in Analogie zu B-Zellen, die durch Anti-IgM aktiviert werden, können Makrophagen durch IgG und AntiIgG aktiviert werden.

Wegen der wesentlich erleichterten Phagozytose sind auch Konjugate aus Anti-Makrophagen/Monozyten-Antikörpern mit Fibronectin und/oder cyto/heterophilen Antikörpern geeignet.

Die hier zur spezifischen Aktivierung von Makrophagen beschriebenen Konjugate können auch für andere Effektorzellen (NK- und CTL-Zellen) eingesetzt werden, wobei dann jeweils die Makrophagen/Monozyten-spezifischen Antikörper entsprechend mit NK- bzw. CTL-bindefähigen Antikörpern im Konjugat ersetzt werden.

In einer weiteren Ausführungsform der Erfindung erfolgt die Stimulierung der Effektorzellen durch Kopplung von einigen, im Prozeß der Zellaktivierung direkt beteiligten bzw. diese simulierenden Verbindungen an Antikörpern mit Spezifität für diese Effektorzellen. Bekanntlich werden bei der T-Zell-Aktivierung zwei biologisch aktive Metabolite aus dem Membran-Inositol gebildet: das Inositol-Triphosphat und das Diacylglycerol. Der erste Metabolit erhöht cytoplasmatisches Ca²⁺ durch Ca-Mobilisierung aus dem Depot im endoplasmatischen Retikulum. Das Diacylglycerol dagegen aktiviert das Enzym Protein-Kinase C. Hinzu muß das Signal 2 (IL-1) treten, um eine T-Zell-Aktivierung zu ermöglichen. Mit Ca-Ionophoren, die das intrazelluläre Ca²⁺ direkt erhöhen sowie mit Phorbol-Estern, die die Protein-Kinase C aktivieren, kann eine T-Zell-Aktivierung sogar ohne Signal 2 (IL-2) erfolgen, da Phorbol-Ester zugleich eine Transmission des Aktivierungssignals ins Zellinnere ermöglichen. Um eine selektivere Effektorzell-Aktivierung zu erzielen, ist es bevorzugt, Inositol-Triphosphat, Diacylglycerol, Ca-Ionophore und/oder Phorbol-Ester an Antikörper mit Spezifität für Effektorzellen zu koppeln.

Eine Stimulierung der Effektorzellen kann auch durch Kopplung von Lysophosphatiden an Effektorzell-spezifische Antikörper erreicht werden, wobei Lysophospholipide direkt mit Antikörpern mit Spezifität für Effektorzellen konjugiert werden, wobei z.B. MPB-PE(N-(4-(p-maleimidophenyl)butyryl)phosphatidylethanolamin als Kopplungsmittel verwendet werden kann.

Wie bei den Suppressorzellen beschrieben, können die CTL durch "Markierung" mit spezifischen Antikörpern bzw. deren Gemischen und durch Kopplung an mitogenen Antikörper oder an mitogene Lektine selektiv stimuliert werden.

Eine bevorzugte Variante des erfindungsgemäßen Arzneimittels enthält die Komponente A, gegebenenfalls mit Anti-CD11b-AK zur Eliminierung der Suppressorzellen und/oder der Inducer der Suppressorzellen mit gleichartig konjugierten Anti-CD45R-AK und als Komponente B mitogene AK (subdosierte Anti-CD3- oder Anti-TCR-1/WT31-AK) oder mitogene Lektine (PHA), konjugiert an die Anti-Leu19-AK, womit cytotoxische T-(und NK-)Subpopulationen selektiv zur Proliferation angeregt werden.

Eine selektive Stimulierung kann auch durch die Quervernetzung muriner Mab mit Anti-Maus-Ak oder deren Untereinheiten erfolgen, wenn ein AK mitogene Eigenschaften (z.B. Anti-CD3-AK) aufweist. Anstelle von mitogenen Mab können mitogene Lektine bzw. deren proteolytische Spaltprodukte treten. Biotin: Avidin-Konjugate bzw. Biotin:Anti-Biotin-Ig-Konjugate können durch Ag:Ak-Konjugate ersetzt werden; in Frage kommen Konjugate mit (inaktivierten) Enzymen einerseits und mit den Anti-Enzym-Ig andererseits Beispiele: Konjugate mit inaktivierter G-6-PDH einerseits und mit dem Anti-G-6-PDH-Ig andererseits, oder Konjugate mit inaktivierter (Meerrettich)-Peroxidase einerseits und mit dem Anti-Peroxidase-Ak andererseits. Die Brückenbildung kann auch über Plasma-Proteine, gekoppelt mit dem 1. Mab, und den entsprechenden Ak, gekoppelt mit dem 2. Mab, erfolgen. Beispiele sind Konjugate des 1. Mab mit humanen IgG oder IgM oder Albumin oder (inaktiv́iertem) Ceruloplasmin, kombiniert mit den Konjugaten des 2. Mabs mit dem jeweiligen antigenspezifischen Ak bzw. Gammaglobulin.

In einer alternativen Variante werden die beiden kooperierenden Zellen - Makrophagen (APC) und T4- bzw. T8-Zelle - durch Heterokonjugate von Anti-CD15/Leu-M1-AK; Anti-CD14/Leu-M3-AK oder Anti-CD11c/Leu-M5-AK mit Anti-CD5/Leu1/T1-AK oder Anti-CD2/Leu5b/T11-AK mit Antigen, z.B. Tumorantigen, seinem Anti-Idiotyp-AK und/oder Anti-CD3/Leu4/T3-AK bzw. Anti-TCR-1/WT31-AK kombiniert, um T4- bzw. T8-Zellen zu stimulieren.

Für die Antigen-spezifische Immunstimulierung kann eine Kombination von Heterokonjugaten aus Antigen bzw. seinem Anti-Idiotyp-AK und dem entsprechenden Ig des IgM-Isotyps mit Heterokonjugaten aus Antigen bzw. seinem Anti-Idiotyp-AK und Anti-CD4/Leu3/T4-AK (zwecks T4-Stimulierung) oder Anti-CD8/Leu2/T8-AK (zwecks Stimulierung), verwendet werden. Analog hierzu kann zur unspezifischen/polyklonalen Aktivierung der T4- und der T8-Zellen eine Kombination von Heterokonjugaten aus Anti-CD3/Leu4/T3-AK oder Anti-TCR-1/WT31-AK und dem, gegen diesen AK gerichteten Ig der IgM-Klasse mit Anti-CD3/Leu4/T3-AK oder mit Anti-TCR-1/Wt31-AK verwendet werden.

Für alle diese Konjugate, die oben beschrieben sind, gilt, daß das Antigen (Tumorantigen) im Konjugat eine potenzierte immunstimulatorische Wirkung erwarten hat, wenn es zuerst epitopschonend enzymatisch oder chemisch zu Einheiten von mindestens 6-10 Peptid(Aminosäure)- bzw. Monosaccharid-Bausteine abgebaut wird und diese Antigen-Bausteine einzeln mit der gleichen zweiten Komponente des Heterokonjugats gekoppelt werden. Hierdurch werden mehrere Klone von T4- bzw. T8-Zellen "angesprochen" und mittelbar (via T4-Zellen) auch mehrere B-Zellen zu Plasmazellen aktiviert. Diese enzymatische "Vorverdauung" simuliert das Prozessieren des Antigens durch den Makrophagen.

Wenn Komponente B des erfindungsgemäßen Arzneimittels in vitro, d.h. an aus Patientenblut gewonnenen Lymphozyten-Kulturen, verwendet wird, kann eine höhere Spezifität durch in vitro Präaktivierung der Patienten-Effektorzellen in Anwesenheit der inaktivierten unveränderten oder chemisch und/oder enzymatisch vorbehandelten Tumorzellen (Membranfragmente, 3MKCl-Tumorextrakte) erzielen, während die Coaktivierung der Suppressorzellen bei einer Hydrolase-Vorbehandlung der Effektorzellen nicht zu erwarten ist. Sollte sie doch vorkommen, so könnten die Suppressorzellen und/oder deren Präkursoren (Inducer-Suppressorzellen) wie oben beschrieben, eliminiert werden.

Bei der Aktivierung der Effektorzellen mit IL-2 kann dessen Wirkung durch Zusatz von Hydrolase verstärkt werden.

Für die in vivo Anwendung von Komponente B können orale Enzympräparate verabreicht werden, die nach der Resorption im Serum proteolytisch wirken, wie es von deren Anwendung für den Abbau von Immunkomplexen bei Autoimmunkrankheiten her bekannt ist.

Die Effektorzellen können auch durch fusogene Substanzen in sub-immunogener Dosierung und/oder durch Substanzen, die die Effektorzellen (a) durch den Einfluß auf das elektrokinetische (Zeta)potential (b) durch den Einfluß auf die Hydratationsenthalpie der Membran-Lipide und/oder (c) durch den Einfluß auf die dielektrische Konstante (DK) aktiviert werden. Diese Substanzen intensivieren den Effektorzell:Zielzell-Kontakt mit der Folge der Aktivierung der Effektorzelle und der Mobilisierung zusätzlicher Lymphozyten-Klone, vor allem T4- und T8-Zellen.

Geeignet sind für diese Variante z.B. Polyethylenglykol (PEG), Polyvinylpyrrolidon (PVP), bevorzugt mit mittleren und höheren Molekulargewichten, Polyvinylalkohol (PVA), Polyvinylacetat (PVAc), Hydroxyethylstärke, Dextran und seine Derivate, Polyalkohole (Polyole), Mannitol, Sorbitol, Fettsäuren und ihre Derivate, Substanzen mit fusogenen und/oder oben präzisierten physikalisch/chemischen Eigenschaften generell.

Die Verwendung von PVP und PEG ist vor allem vorteilhaft bei der in vitro-Aktivierung, da diese Polymere ausgezeichnete Kryoprotektanten sind, deshalb werden sie allein oder vorteilhaft mit DMSO (8 bis 20 %) zur Einfrierung und Konservierung von Zellen, z.B. Leukozyten, sowie zur Erhaltung der Aktivität von (präaktivierten) Effektorzellen erfindungsgemäß bevorzugt. Der präaktivierte Zustand der Effektorzellen konnte nach Einfrieren nur dann voll erhalten bleiben, wenn PVP oder PEG zu DMSO zugesetzt worden waren.

Es ist vorteilhaft, generell anstelle von oder zusätzlich zu den Antigenen (Tumorantigenen) in den einzelnen (Hetero)konjugaten deren enzymatische oder chemische Abbauprodukte und/oder die durch eine doppelte Immunisierung, d.h. eine sukzessive Immunisierung in zwei verschiedenen Spezies gewonnenen Anti-Idiotyp-AK bzw. vorteilhaft deren Fab(F(ab')₂-Untereinheiten in den (Hetero)konjugaten zu verwenden. Bevorzugt sind solche Antigen-Spaltprodukte kurze Peptide mit ca. 6 bis 10 Aminosäure-Einheiten oder Oligosaccharide mit ebenso ca. 6 bis 10 Monosaccharid-Grundbausteinen, bzw. Glykopeptide gleicher Größe, abhängig von dem Aufbau des Antigens (Tumorantigens: TATA, TSTA, TSA...). Dies ahmt die natürlichen Verhältnisse nach, wo jedes Antigen, einschließlich Tumorantigen, zuerst in APC (Makrophagen) "prozessiert", d.h. durch die (lyosomalen) Hydrolasen des Makrophagen in kleinere Bausteine, z.B. Peptide, gespalten werden muß, um dann in Assoziation (Kontext) mit MHA II-Antigenen an die Helfer-T4-Zelle "präsentiert" zu werden und diese (zusammen mit dem Monokin IL-1) zu aktivieren.

Es ist bevorzugt, in Komponente B zusätzlich zu den beschriebenen Heterokonjugaten neben dem "Signal 2" der Helfer- T-Zell-Aktivierung, dem IL-1, zwei monoklonale Antikörper (bzw. deren Fab/F(ab')₂-Untereinheiten) und zwar Tp67-Mab (entsprechend dem CD5/Leu1/T1-Mab) und/oder Tp44, gegebenenfalls kombiniert mit IL-1 oder IL-2 einzusetzen, um die Wirkung der einzelnen (Hetero)konjugate weiter zu verbessern. Diese Mab zeigen, analog zu IL-1, eine synergistische Wirkung bei der Aktivierung von T(T4)-Zellen. Sie können sowohl bei der unspezifischen/ polyklonalen als auch bei der (tumor)spezifischen T-Zell-Aktivierung eingesetzt werden. Diese Mab können als Adjuvans dienen; sie binden sich selektiv an T-Zellen und aktivieren nur jene T-Zellen, die das (Tumor)antigen "tragen". Hierdurch kann eine antigen-spezifische Immunstimulation erzielt werden, bei der die Kenntnis des (Tumor)antigens nicht vorausgesetzt wird.

Es können aber auch Heterokonjugate hergestellt werden, deren eine Komponente den beiden Mab(Tp67 und Tp44) bzw. deren Fab(F(ab')₂-Untereinheiten entspricht und deren zweite Komponente entweder aus Mab (bzw. Mab-Untereinheiten) gegen CD3 oder Ti-Rezeptor besteht (polyklonale T-Zell-Aktivierung) oder aber aus dem (Tumor)antigen bzw. seinen Spaltprodukten und/oder dem entsprechenden Anti-Idiotyp-Ig (bzw. dessen Fab/F(ab')₂-Untereinheiten) aufgebaut ist (monoklonale, antigen-spezifische T-Zell-Aktivierung).

Allgemein gilt, daß je nach Situation (Eliminierung von Suppressorzellen oder Stimulierung von Leukozyten-Subpopulationen) die in den einzelnen Heterokonjugaten vorgesehenen AK von einem Isotyp bzw. von einer Ig(sub)klasse sein müssen, die entweder zur RES-Eliminierung (z.B. bei der Suppressorzell-Eliminierung) oder nur zur Zellaktivierung ohne eine RES-Eliminierung (z.B. bei der spezifischen und unspezifischen T-Zell-Aktivierung) führt. Beispiel solcher Diskriminierung zwischen RES-eliminierbaren und -nichteliminierbaren AK ist der Anti-Lyt1-AK, gerichtet gegen eine Subklasse muriner T-Lymphozyten (entsprechend dem humanen Anti-CD5-AK): während der Ig2b-Isotyp des gleichen AK zur Lyse und RES-Eliminierung entsprechender T-Zellen führt, wirkt sich der Ig2a-Isotyp in einer Aktivierung ohne Zellyse bzw. Zelleliminierung aus.

Zusätzlich zu den Komponenten A und B kann das Arzneimittel noch zur Verstärkung der Effektorzell-Aktivierung Heterokonjugate enthalten, bestehend aus verschiedenen Cytokinen wie IL-1; IL-2; IL-3; G-CSF und MG-CSF. Bevorzugt werden dazu Heterokonjugate von IL-1 mit IL-2, IL-3 oder G(MG)-CSF sowie Heterokonjugate von IL-2 mit IL-3; M-CSF; MG-CSF eingesetzt. Ebenso geeignet sind Konjugate von Interferon mit IL-1, IL-2 und/oder IL-3, M-CSF, MG-CSF oder auch mit Mitogenen (mitogenen Antikörpern oder mitogenen Lektinen bzw. deren Untereinheiten) bei polyklonaler oder mit dem Antigen bzw. seinem Anti-Idiotyp-Antikörper zur monoklonalen T-Zell-Aktivierung. Der besondere Vorteil eines Interferon-haltigen Konjugates ist die gezielte Antigenitätssteigerung durch Post-Expression von MHC II(HLA-DR)- und/oder MHC I (HLA-A,B,C)-Antigenen.

Über die AIDS-Problematik sind zahllose Arbeiten erschienen, u.a. in den Zeitschriften wie AIDS-Forschung (AIFO) (Herausgeber: R. Hehlmann)/Schulz-Verlag München, ferner in AIDS Research and Human Retroviruses (Herausgeber: D.P. Bolognesi)/M.A. Liebert Publishers New York, sowie in Nature und Science. Allgemein wird die AIDS-Problematik in "Basic & Clinical Immunology" (P. Stites, J.D. Stobo, J.V. Wells, editors) Appleton & Lange Publ. Norwalk (1987) abgehandelt. Möglichkeiten zur Therapie sind dem Übersichtsartikel "Strategies for antiviral therapy in AIDS (von H. Mitsuya und S. Broder), Nature 325:773 (1987) zu entnehmen.

Bis heute gibt es jedoch noch keine Medikamente, die zur Heilung von AIDS führen würden und auch nicht Impfstoffe, mit denen gegen AIDS prophylaktisch geimpft werden könnte. Die meisten bisher vorgeschlagenen Medikamente konzentrieren sich auf eine Hemmung der HIV-Replikation, so auch das bereits zugelassene Medikament AZT.

AIDS-, aber auch ARC- und LAS-Patienten weisen eine fatale Immundefizienz bzw. eine fatale immunsuppressive Lage, die noch gravierender als bei Tumorpatienten ist, auf, die u.a. auch die Ausbreitung von normalerweise harmlosen opportunistischen Infekten, aber auch die Entstehung von ansonsten sehr seltenen Tumorarten (Kaposi-Sarkom) regelrecht fördern bzw. überhaupt ermöglichen. Erfindungsgemäß werden nun Arzneimittel zur Verfügung gestellt, die dieses Grundmanko beheben und durch Eliminierung der HIV-protektiven Suppressorzellen und Reaktivierung der Patienten-Immunabwehr eine Wende der Krankheit herbeiführen, da die HIV nicht nur in ihrer Replikation gehemmt, sondern aktiv eliminiert werden.

Am schwierigsten ist dabei der HIV-Befall von Nerven- und Gehirnzellen zu bekämpfen, was mit dem erfindungsgemäßen Arzneimittel gelingt, da die restlichen Tumorzellen bzw. Infektträger durch die eigene Immunabwehr "aufgespürt" und eliminiert bzw. unter Kontrolle gehalten werden, wenn die protektiven Suppressorzellen (weitgehend) eliminiert sind und die Effektorzellen prä- oder reaktiviert werden.

Dadurch kann es zur kritischen Wende kommen, die oft auch durch reine quantitative Verhältnisse der einzelnen Leukozyten-Subpopulationen zueinander (z.B. dem T4/T8-Quotienten oder das Verhältnis von CTL zu Suppressor-Subpopulationen) bestimmt wird.

Das erfindungsgemäße Arzneimittel wirkt in drei Phasen:
(a) der Phase Iₒ: Eliminierung der HIV, gegebenenfalls mit Komponente C;
(b) Phase I: Eliminierung/Coeliminierung der HIV-protektiven Suppressorzellen mit Komponente A und
(c) Phase II: in vitro Präaktivierung bzw. in vivo Reaktivierung der HIV-überwachenden und eliminierenden Effektorzellen mit Komponente B.

Phase Iₒ und Phase I werden zum Teil von Komponente A allein bewirkt, teilweise ist die Verwendung einer dritten Komponente C für die Phase Iₒ notwendig. Die beiden Phasen I (Eliminierung der Suppressorzellen) und II (Prä- bzw. Reaktivierung der Effektorzellen) verlaufen im Prinzip analog zu den beiden bereits beschriebenen Phasen I und II bei der Tumor-Behandlung sind, so daß im folgenden nur noch die die Unterschiede zu der Behandlung von Tumorpatienten besprochen werden, während ansonsten auf die detaillierte Beschreibung dieser gemeinsamen Phase I und II bei Tumorpatienten verwiesen wird.

Die potentiellen HIV-Träger (T4-Zellen, aber auch T8-Zellen, B-Zellen, Makrophagen/Monozyten) werden mit entsprechenden Antikörpern opsonisiert und RES-eliminiert bzw. coeliminiert, unabhängig davon, ob sie HIV-infiziert sind und ob sie die viralen Strukturen (HIVenv-Glykoproteine) an der Zelloberfläche coexprimieren.

Alle hier beschriebenen Präparate können mit Anti-HIV-Medikamenten (AZT und anderen, z.B. in der eingangs zitierten Publikation von Mitsuya und Broder einzeln aufgeführten Virustatika und anderen AIDS-Mitteln) kombiniert werden.

Zur Eliminierung von HIV in den T4-Zellen als den bevorzugten HIV-Wirtszellen sowie in T8-Zellen, B-Zellen und Makrophagen/Monocyten können die folgenden Antikörper und Antikörper-Kombinationen verwendet werden, die teilweise in Komponente A bereits enthalten sind: Anti-CD4/Leu3/T4-AK und/oder Anti-CD3/Leu4/T3-AK in Kombination mit Anti-CD8/Leu2/T8(T5)-AK, Anti-CD19/Leu12-Mab, Anti-CD22/Leu14-Mab, Anti-CD20/Leu16-Mab, Anti-CD21/CR2-Mab; Anti-CD15/LeuN1-Mab, Anti-CD14/LeuM3-Mab oder Anti-CD11c/LeuM5-Mab sowie Anti-Leukozyte/HLe-1-Mab (ein pan-Leukozyten-AK) oder Anti-Lymphozyten/Thymozyten-Serum bzw. daraus gewonnene Globuline, nach der Art von ALS/ALG oder ATS/ATG. Diese Antikörper können außerdem noch mit AK gegen virale env-Glykopeptide, z.B. Anti-gp120/160-Ig und gegen das Kernmaterial von HIV-befallenen Zellen (standardisierbar z.B. durch Immunisierung mittels HIV-infizierte, mit Detergenzien, Ultraschall oder Schockgefrierung lysierte H9- oder ATH8-Zellen) kombiniert werden.

Die Eliminierung von HIV-Wirtszellen kann analog der Eliminierung von Suppressorzellen (im Rahmen der Phase I der Tumorbehandlung) gesteigert werden.

Eine spezielle weitere Möglichkeit der Effizienzsteigerung bei der Eliminierung von HIV-Wirtszellen besteht in der wiederholten Injizierung von allogenen T-Zellen, die in einer Art MLC/MLR in vitro gegen Patienten-T-Zellen allopräaktiviert wurden.

Nachdem die Eliminierung der HIV-protektiven Suppressorzellen im Prinzip in allen Punkten jener der tumorspezifischen Suppressorzellen entspricht, können hier dieselben Wirkstoffe für Komponente A verwendet werden. Wichtig ist dabei, daß die Phase Iₒ und die Phase I, wie erwähnt, zu einer einzigen Phase (Phase I) zusammengezogen werden können und die AK und AK--Kombinationen gleichzeitig beide Funktionen, die HIV-und die Suppressorzell-Eliminierung übernehmen können. Dies gilt für alle oben (unter Phase Iₒ) beschriebenen Substanzen und Substanzgemische (AK und AK-Kombinationen), außer für die alleinige oder allein mit Mab gegen B-Zellen und/oder gegen Makrophagen/Monozyten kombinierte Anwendung von Anti-CD4/Leu3/T4-Mab.

Auch für Phase II, in vivo Präaktivierung bzw. direkte in vivo Reaktivierung der Effektorzellen, können für Komponente B im wesentlichen die beschriebenen Verbindungen verwendet werden.

Zusätzlich können hier jedoch bei der Präaktivierung der autologen Effektorzellen in vitro virustatisch wirkende Präparate, wie AZT, der Zellkultur zugesetzt werden, wobei in vitro deren Dosis und hiermit Wirksamkeit höher als sonst in vivo üblich gewählt werden können.

Zur Erzielung einer höheren Spezifität der prägenerierten Effektorzellen ist sowohl in vitro als auch in vivo die gleichzeitige Gabe von inaktivierten, chemisch oder enzymatisch veränderten, möglichst attenuierten HIV, bevorzugt fixiert an H9- oder ATH8-Zellen geeignet. Zur Mobilisierung weiterer kreuzreagierender Lymphozyten-Klone ist es möglich, die inaktivierten, zum Teil modifizierten HIV mit SIV, optimal jene, die nur 10 bis 30%ige Homologie bzw. Kreuzreaktivität mit HIV aufweisen, zu kombinieren und beide, die inaktivierten HIV und SIV, möglichst auf verschiedene, bzw. durch chemische oder enzymatische Vorbehandlung "xenogenisierte" Trägerzellen zu fixieren.

Um auch eine Prägenerierung von Effektorzellen, z.B. CTL, die spezifisch gegen das für AIDS/ARC/LAS-Patienten typische Muster an opportunistischen Infekten gerichtet sind, während des Aufbaus der patienteneigenen Immunabwehr in der Phase II zu ermöglichen, wird bevorzugt mittels eines standardisierbaren Antigen-Gemisches (z.B. eines Lysats aus den Vertretern der opportunistischen Infekte) unabhängig von aktuellen Infekten des Patienten, in vitro oder in vivo immunisiert.

Was für das Tumorantigen, seine Spaltprodukte und entsprechende Anti-Idiotyp-Ig als Bestandteile der (Hetero)konjugate gilt, gilt auch für die HIV-Antigene, speziell die immunologisch relevanten env(gp120/160/110/130)-Glykoproteine.

Bei AIDS-Patienten wurden Antikörper und cytotoxische T-Zellen nicht nur gegen env-, sondern auch gegen gag- und pol-Genprodukte und Antikörper gegen virale Enzyme und regulatorische Proteine nachgewiesen. Daher können Heterokonjugate aus viralen Antigenen, deren Spaltprodukten und den entsprechenden Anti-Idiotyp-Ig bzw. deren Fab(F(ab')₂)-Untereinheiten mit Antikörpern (bzw. deren Untereinheiten gegen CD3, Ti-Antigen-Rezeptor, CD2, Tp67 (CD5) oder Tp44 verwendet werden. Dabei sind unter "viralen Antigenen" alle, auch core-Antigene von HIV zu verstehen, wobei env-Glykopeptide bevorzugt sind.

Alle anderen (Hetero)-Konjugate und sonstigen Maßnahmen zur T-Zell-Aktivierung sind analog wie bei der Tumorbehandlung beschrieben. Der einzige Unterschied ist der, daß das Tumorantigen, bezeichnet als "Antigen" und sein Anti-Idiotyp-AK in den Heterokonjugaten durch virale Antigene, speziell die env-Glykopeptide und ihre Anti-Idiotypen ersetzt werden.

Eine HIV-spezifische Immunisierung kann mit viralen Peptiden, die an verschiedene, nicht-kreuzreagierende Trägerproteine gekoppelt sind, erzielt werden.

Das Prinzip ist die Durchbrechung einer eventuellen restlichen Toleranz gegenüber HIV-Strukturen nach der Suppressorzell-Eliminierung durch Kopplung der HIV-Antigene, primär der env-Glykopeptide, an stark immunogene Trägerproteine, wobei die Kreuzreaktivität des Gesamtmoleküls (gleiches Hapten auf differenten Trägern) optimal 15 bis 25 % betragen und nicht 75 % übersteigen sollte. Die viralen Antigene können durch entsprechende Anti-Idiotypen ersetzt werden. Diese Hapten-Carrier-Konjugate können standardisiert werden. Sie können mit Antikörpern gegen toleranzsuszeptible CD1(T6)- und CD38(T10)-positive T-Zell-Präkursoren kombiniert werden.

Derartige Konjugate eignen sich sowohl für die Prophylaxe als auch für die Therapie von AIDS/ARC/LAS.

Niedermolekulare CD4-Fragmente und Fragmente des gp120 (gp160)-Moleküls, enthaltend 10 bis 20 Aminosäurereste, können die HIV-Interaktion mit dem CD4-Rezeptor hemmen, ohne dabei immunogen zu sein. Hierdurch kann eine Neutralisierung des therapeutisch einzusetzenden CD4-Moleküls verhindert werden. Diese Fragmente könnten gentechnologisch hergestellt werden.

Diese Hemmung hat die gleiche Basis wie die Elution des makromolekularen Ligands durch Mono- und Oligomeren in der Affinitätschromatographie bzw. die inhibierte Lektinbindung an Glykoproteine in Anwesenheit des spezifischen Monosaccharids.

Derartige gp120 (gp160)-Fragmente eignen sich sowohl für die Prophylaxe als auch für die Therapie von AIDS/ARS/LAS.

Ebenso kann die Bindung von HIV and CD4 durch niedermolekulare, nicht-immunogene HLA-DR-Untereinheiten gehemmt werden. Das HLA-DR-Molekül ist der direkte Ligand des CD4-Rezeptors. Seine niedermolekularen, nicht-immunogenen Fragmente konkurrieren mit dem HIV um den CD4-Rezeptor. Man kann eine Voranreicherung der mit CD4 interagierenden HLA-DR-Fragmente durch eine CD4-Chromatographie erzielen. Zwischen gp120 (gp160) und HLA-DR muß es Homologien geben, da beide mit dem CD4-Molekül interagieren; auch zwischen gp41 und HLA-DR wird Homologie diskutiert. Auch diese HLA-DR-Fragmente eignen sich für die Prophylaxe und Therapie von AIDS/ARC/LAS.

Fab/F(ab')₂-Untereinheiten von Anti-CD4-Antikörper konkurrieren mit dem HIV um den CD4-Rezeptor. Ihr niedrigeres Molgewicht (50 kD) macht sie weniger immunogen und läßt die Passierung der Hirn-Blut-Schranke erwarten. Sie eignen sich für die Prophylaxe und Therapie.

Eine Bindung von freien HIVs und freien env-Glykopeptiden ist im Plasma durch subdosierte Anti-HLA-DR-Antikörper möglich. Durch subdosierte Anti-HLA-DR-AKs und/oder ihre Fab/F(ab')₂-Untereinheiten könnten freie HIVs und freie env-Glykopeptide im Plasma "neutralisiert" werden, da Homologien zwischen HLA-DR und env-Glykopeptiden (gp120 und/oder gp41) bestehen. Nachdem Homologien zwischen HIV- und anderen retroviralen env-Glykopeptiden einerseits und IL-2 berichtet worden sind und die immunsuppressive Wirkung von Retroviren auf diese Homologie zurückgehen dürfte, ist es bevorzugt, subdosierte Anti-HLA-DR-AKs mit IL-2 zu kombinieren. Lösliches HLA-DR wirkt z.U. von dem membrangebundenen HLA-DR hemmend auf die Immunreaktion.

In einer bevorzugten Ausführungsform werden die oben beschriebenen Heterokonjugate, insbesondere die, die T-Zellen abhängig von APCs aktivieren, mit IL-1 und/oder IL-2 kombiniert. Zur weiteren Effizienzsteigerung können noch mitogene Antikörper und/oder Lektine (PHA) sowie Immunmodulatoren (BRM) zugesetzt werden.

Syncytia-Bildung kann durch Konjugate aus antiviralen Antikörpern mit stark polaren, sauren oder basischen Gruppen durch hohes Zetapotential Konjugat-beladener Zellen verhindert werden.

Heterokonjugate aus HIV-spezifischen Antikörpern und aus mitogenen Antikörpern (Anti-CD3-, Anti-Ti-, Anti-CD2-, evtl. Anti-CD5-AKs) vereinen hohe Spezifität mit polyklonaler Aktivierung, da die Bindung eines solchen Heterokonjugats an die viralen Membranstrukturen der infizierten Zelle die Bindung und Aktivierung von T-Zellen in antigenunspezifischer und MHC-nichtrestringierter Weise ermöglicht.

Die äußere und innere Lipidmembran von HIV kann durch hydrophile Polymere mit partiell lipophilem Charakter geschädigt werden. Nachdem die Zunahme der Cholesterollöslichkeit in wäßrigen Lösungen von Polymeren wie PVP und PEG nachgewiesen werden konnte, werden solche Polymere, von denen einige (PVP, Hydroxyethylstärke) als Plasmaexpander Anwendung finden, zur Inaktivierung von Plasma-HIV eingesetzt.

Es ist bekannt, daß die Effizienz der Eliminierung von zirkulierenden Immunkomplexen (CIC) in hohem Maße von ihrer Zusammensetzung sowie ihrem Molgewicht abhängt. Es sollte daher das virale Antigen im CIC durch entsprechenden Anti-Idiotyp-Antikörper ersetzt werden; sein Fc-Teil könnte die Phagozytose durch Makro- und Mikrophagen beschleunigen. Ein zusätzlicher Effekt könnte durch Hitzeaggregierung oder chemische Prädenaturierung des Anti-Idiotyps bzw. durch seine Kopplung an Staphylococcus-Protein A erzielt werden.

In Analogie zu solchen, strukturell und funktionell simulierenden Anti-Idiotypen bei Insulin und Thyreoglobulin könnten statt Cytokinen Anti-Idiotyp-Antikörper, die die einzelnen Cytokine (IL-1; IL-2; IL-3; M-CSF; MG-CSF) strukturell und funktionell "imitieren", verwendet werden. Diese können dann, da in ausreichenden Mengen vorhanden, direkt oder in Form der oben beschriebenen Heterokonjugate eingesetzt werden.

Vorteil der erfindungsgemäßen Arzneimittel ist es, daß sie, verglichen mit dem für Krebs und AIDS bisher verwendeten Mittel minimale Nebenwirkungen haben. Von den zahlreichen Studien mit monoklonalen und polyklonalen heterologen Antikörpern (z.B. OKT3 oder ATG), die bei Patienten mit Nieren-Transplantaten klinisch eingesetzt wurden, ist bekannt, daß die Nebenwirkungen minimal sind und jenen bei grippalen Infekten entsprechen. Zum Unterschied von Cytostatika haben die verwendeten Präparate praktisch keine Langzeit-Nebenwirkungen.

Die eliminierten Lymphozyten werden innerhalb von Tagen nach Absetzen der Therapie aus den Präkursorzellen nachgebildet. Das Problem der Neutralisierung von heterologen Antikörpern wird umgangen.

Die HIV werden unabhängig davon, ob sie im Zellgenom bereits integriert sind oder ob sie sich noch im Cytosol befinden, mit den Trägerzellen in den RES-Organen effizient mit abgebaut; die RES-Makrophagen, wie z.B. die Kupfferschen Sternzellen in der Leber, sind auf einen schnellen Abbau der gealterten und opsonisierten Leukozyten spezialisiert; die lysosomalen DNAsen bzw. RNAsen der RES-Makrophagen unterscheiden nicht zwischen der zellulären und der viralen DNA bzw. RNA.

Selbst bei der Miteliminierung von Makrophagen/Monozyten oder bei der Eliminierung von Leukozyten, z.B. mit dem erwähnten Anti-Leukozyte/HLe-1-Mab bzw. dem ALS/ALG handelt es sich um eine Behandlung, die z.B. wesentlich schonender für den Patienten ist als z.B. die Ganzkörperbestrahlung oder die hochdosierte Chemotherapie bei Leukämiepatienten, weil hier die Stammzellen und die frühen Präkursorzellen weitgehend erhalten bleiben und zur baldigen Nachreifung der eliminierten Leukozyten führen.

Die für die erfindungsgemäßen Arzneimittel verwendeten Antikörper, die gegen spezielle Oberflächen-Antigene von T- bzw. B-Zellen gerichtet sind, sind im Handel erhältlich und können aus hinterlegten Zellinien gewonnen werden. So wird Anti-CD4-AK von der Zellinie ATCC CRL 8002; Anti-CD3-AK von der Zellinie ATCC CRL 8001; Anti-CD8-AK von der Zellinie ATCC CRL 8014, 8013 oder 8016; Anti-T10-AK von der Zellinie ATCC CRL 8022; Anti-T11-AK von der Zellinie ATCC CRL 8027; Antitransferin-Rezeptor-AK von der Zellinie ATCC CRL 8021, Anti-Leu15- bzw. Anti-CD11b-AK von der Zellinie ATCC CRL 8026 und Anti-T1-AK von der Zellinie ATCC CRL 8000.

Erfindungsgemäß wird auch ein Verfahren zur Behandlung von Autoimmunkrankheiten zur Verfügung gestellt.Autoimmunkrankheiten gehen auf autoaggressive T- und/oder B-Zellen zurück. Sie sind Ausdruck einer gestörten self-Toleranz; die autologen Lymphozyten "verwechseln" Eigenstrukturen (Autoantigene) mit Fremdantigenen und entwickeln dagegen eine destruktive zelluläre und/oder humorale Immunreaktion.

Bei der Behandlung der Autoimmunkrankheit mit Immunsuppressiva auf Corticosteroid- oder Cytostatika-Basis besteht ein wesentliches Problem darin, daß beide Klassen von Immunsuppressiva, Corticosteroide (Prednison, Prednisolon) und Chemotherapeutika (Azathioprin, Methotrexat, Cyclophosphamid) nur proliferierende autoaggressive Zellen erfassen, während die hoch aktiven reifen, enddifferenzierten T- und/oder B-Zellen weitgehendst verschont bleiben.

Deshalb werden erfindungsgemäß durch spezifische AK und deren Konjugate diese refraktären, reifen autoreaktiven T- und/oder B-Zellen eliminiert (Phase I) und zur Verhinderung künftiger Rezidive in vitro oder in vivo autoantigenspezifische Suppressorzellen generiert und klonal expandiert (Phase II).

In Phase I werden autoaggressive T- und/oder B-Zellen eliminiert. Diese Eliminierung refraktärer autoreaktiver T- und/oder B-Zellen, deren Zellteilung abgeschlossen ist, geschieht mit spezifischen AKs in freier oder konjugierter Form. Die Konjugate bestehen aus AK und Cytotoxin ("Immuntoxin"), Radionuklid oder Cytostatika.

In Phase II werden zur Rezidivverhinderung autoantigenspezifische Suppressorzellen in vitro oder in vivo generiert. Diese Suppressorzellen sollen die Entstehung und Aktivität von autoaggressiven Klonen verhindern oder hemmen. Bei den Autoantigenen - soweit bekannt - handelt es sich oft um Strukturen, die im homologen System weitgehendst identisch oder histokompatibel und folglich auch standardisierbar sind. Zum Teil handelt es sich um Mikroorganismen-Antigene, die durch ihre Ähnlichkeit mit Autoantigenen autoreaktive Klone aktivieren und die Krankheit auslösen ("antigenic mimicry"). Auch diese Antigene sind

Da die autoaggressiven T-Zellen und auch ihre Inducers in erster Linie unter den CD4-positiven Zellen zu suchen sind, reicht nach dem einfachsten Therapieschema bereits die alleinige Anwendung von Anti-CD4/T4/Leu3-AK aus. Ein günstiger Nebeneffekt ist die Verhinderung der Neutralisation angewandter xenogener Antikörper.

Allgemein können neutralisierende Antikörper durch Zusatz von diesen Anti-CD4- und/oder Anti-CD19- bzw. Anti-CD20- bzw. Anti-CD22-AK verhindert werden. Geeignet sind auch Kombinationen von Anti-CD4/T4/Leu3-AK mit Anti-CD8/T8/Leu2-AK; Anti-CD8- mit Anti-CD3-AK; Anti-CD2/T11/Leu5-AK; Anti-CD5/T1/Leu1-AK oder Anti-CD45/HLe-1-AK sowie Anti-MHC II (HLA-DR/Ia)-AK, Anti-CD25/IL-2(Tac)-Rezeptor-AK, Anti-T9/Transferrin-Rezeptor-AK, Anti-CD38/Leu17/T 10-AK.

Diese Antikörper können allein oder kombiniert verwendet werden. Sie erfassen die aktivierten autoaggressiven Zellen.

Da von einigen Forschern auch NK-Zellen als autoaggressive Zellen bei Autoimmunkrankheiten angesehen werden, können von NK-Zellen durch Anti-CD16/Leu11- und/oder Anti-NKH-1/Leu19-AK eliminiert werden.

Bei Autoimmunerkrankungen spielen die Autoantikörper oft eine große Rolle. Dies geht so weit, daß die Lymphopenie und die absolute und relative T-Zell-Abnahme, sowie der mehrfach berichtete, selektive Suppressor-T-Zell-Mangel als Folge einer Antikörperentwicklung gegen autologe Lymphozyten angesehen wird. Der humorale Teil der Erkrankung wird durch autoreaktive B-Zellen, vor allem deren enddifferenzierte Form, die Plasmazellen, verursacht; diese wiederum brauchen "Hilfe" seitens der T4-Zellen. Zur Eliminierung geeignet sind Anti-CD19/Leu12-und/oder Anti-CD20/Leu16- und/oder Anti-CD22/Leu14-AK. Diese Antikörper können mit Anti-CD4-AK kombiniert werden. Sind auch autoaggressive T-Zellen vorhanden, so werden zusätzlich gegen diese gerichtete Antikörper verwendet.

Die Antikörper können jeweils zur Effizienzsteigerung mit Cytotoxinen wie Ricin-alpha-Kette; Abrin; Diphterie-Toxin/Toxoid; Cytostatika wie Doxorubicin und/oder mit Radioisotopen, z.B. ¹³¹⁽¹²⁵⁾I oder ¹¹¹In konjugiert werden.

Es können auch bei der Behandlung von Autoimmunkrankheiten weitere Heterokonjugate, wie oben bei der Tumor- und AIDS-Therapie beschrieben, eingesetzt werden.

Diese Antikörper und AK-Kombinationen können selbständig oder ergänzend zu den "konventionellen" Therapien appliziert werden; bei der Integrierung in bestehende Therapieschemata müssen diese Antikörper nur die refraktären, corticosteroiden- oder cytostatikaresistenten autoaggressiven Klone eliminieren.

In Phase II werden autoantigenspezifische Suppressorzellen generiert und klonal expandiert.

Ein Antigen wird zum Tolerogen, wenn die Immunisierung unter Immunsuppression erfolgt, weil im zeitlichen Wettlauf die Suppressorzellen einseitig bevorzugt werden und anschließend auf zellulärer und humoraler Ebene die Immunstimulierung hemmen ("down-regulation" der Immunabwehr).

Das jeweilige Autoantigen in standardisierter Form, gegen welches sich die Krankheit richtet (z.B. der Acetylcholin-Rezeptor bei der Myasthenia gravis oder das Thyreoglobulin beim Hashimoto-Thyreoiditis oder das basische Myelin-Protein bei der MS) wird in vitro mit dem Patientenlymphozyten 4 bis 6 Tage lang inkubiert.

Die Immunsuppression wird durch Cyclosporin A, Anti-HLA-DR-Fab(F(ab')₂, Anti-CD4-Fab/F(ab')₂, Anti-NKH-1/Leu19-AK, Corticosteroide (Prednison, Methylprednisolon), Chemotherapeutika (Methotrexat, Azathioprin, Endoxan), PGE2, Anti-Interferon und/oder Anti-Il-2 und/oder ConA(PNA) aufrechterhalten.

Nach der Generierung der autoantigenspezifischen Suppressorzellen können diese (in Abwesenheit von Immunsuppressiva) durch IL-2 klonal expandiert werden. Danach erfolgt die Reinjizierung der so vorbehandelten Patientenlymphozyten.

Suppressorzellen können auch in vivo generiert werden. Dies erfolgt durch immunsuppressive Behandlung der, von autoaggressiven T- und/oder B-Zellen befreiten Patienten.

Autoaggressive T-Zellen können, um sie zu hemmen, auch mit Konjugaten aus IL-2 bzw. aus Anti-CD25-, Anti-T9- und/oder Anti-HLA-DR plus Cyclosporin A bzw. PGE2/PGE1 inkubiert werden.

Mit Konjugaten, bestehend aus Antoantigen bzw. seinen Anti-Idiotyp (Fab/F(ab')₂-Untereinheiten) und Cytotoxin und/oder Tolerogen (z.B. D-GL) werden beteiligte T- und B-Zellen auf hoch-selektive Weise ausgeschaltet.

Alopecia areata hat alle Merkmale einer Autoimmunkrankheit. In den Haarfolikeln beobachtet man Zellinfiltrate, bestehend aus T4- und T8-Zellen, Makrophagen, dendritischen Zellen und HLA-DR-positiven epithelialen Zellen. Typisch ist eine Erhöhung des T4/T8-Quotienten von 2 auf 4 (in Ausnahmefällen auf knapp 20) sowie eine starke Zunahme an HLA-DR-positiven Zellen. Auch in Peripherblut wird während der aktiven Phase eine T4/T8-Verschiebung meßbar. Eine Therapie von verschiedenen Alopezieformen wie alopecia areata, alopecia generalis kann daher ähnlich der Behandlung der Autoimmunerkrankungen (Phase I und Phase II) erfolgen.

Dazu werden dieselben Substanzen und Substanzgemische wie für die Behandlung der Autoimmunkrankheiten eingesetzt, wobei als Autoantigen standardisierbare Zellen, Zellfragmente und (3MKCl)-Extrakte aus dem Haarfollikel-Bereich eingesetzt werden.

Auch hier können direkt beteiligte T- und B-Klone durch Immunkonjugate, bestehend aus Autoantigen bzw. Anti-Idiotyp und Cytotoxin oder/und durch Konjugate aus gleichem Antigen (Anti-Idiotyp) und Tolerogen (D-GL) ausgeschaltet werden.

Weiterhin wird erfindungsgemäß ein Verfahren zur Verbesserung von Impfstoffen geschaffen. Impfstoffe sollen durch Präimmunisierung mit dem lebenden oder toten Krankheitserreger oder seinem Extrakt eine spätere Erkrankung verhindern. Sie sollen ein "immunologisches Gedächtnis" als Schutz gegen den Krankheitserreger aufbauen. Dieses besteht aus "positiven" und "negativen" Gedächtniszellen. Zu den ersteren gehören Helfer- und cytotoxische (CTL) T-Zellen, zu den letzteren die Suppressor-T- und B-Zellen.

Die spätere Immunantwort auf den Krankheitserreger hängt in hohem Maße vom Verhältnis der "positiven" zu den "negativen" Gedächtniszellen ab. Es muß daher im Interesse jeder Impfung sein, durch ein "Hinauszögern" dar Suppressor-T-Zell-Regenerierung eine möglichst hohe Ausbeute an Helfer- und cytotoxischen T-Zellen sowie Plasmazellen zu erhalten, die dann das "immunologische Gedächtnis" prägen. Dieses Ziel soll durch Eliminierung lokal induzierter Suppressorzellen sowie durch Substanzen, die eine Generierung von Suppressorzellen während der Vakzination stark verzögern, erreicht werden.

Die Impfung entspricht dem "priming" gegen das Erreger-Antigen, welches über die Zusammensetzung der Gedächtniszellen entscheidet, während die Nachimpfung dem "boosting", d.h. der klonalen Expansion gebildeter Subklone dient. Durch immunstimulierende Maßnahmen kann die Generierung von Suppressorzellen hinausgezögert und somit die Entstehung möglichst vieler "positiver" und möglichst weniger "negativer" Gedächtniszellen eingeleitet werden.

Bei einer Vakzination, die möglichst i.d. oder s.c. oder i.m. durchgeführt werden soll, um die Makrophagen in möglichst früher Phase in den Immunprozeß zu involvieren, soll die lokale Suppressorzellentstehung mit folgenden Stoffen oder Stoffgemischen verhindert oder verzögert werden:

Antikörper gegen Suppressor-T-Zellen wie Anti-CD45R/Leu18 (2H4, HB10, HB11, 3AC5)-AK); IL-2; IL-1; IL-3; M-CSF; MG-CSF; BRM (MDP; MTP-PE...); Anti-PGE2; PGE2-Hemmer (z.B. Indomethazin); Anti-Histaminika (Histamin-Strukturanaloga; Blocker des Histamin-Rezeptors); Anti-Corticosteroide (z.B. Anti-Hydrocortison); Konjugate aus Erreger-Antigen und D-GL (Copolymere aus D-Glutamat und D-Lysin); Mercaptoethanol; Thioglykollat; Mitogene (subdosierte mitogene Antikörper und/oder mitogene Lektine); Enzyme (Hydrolasen, wie Lysozym, Neuraminidase/Sialidase, Hyaluronidase, spezielle Proteasen und Lipasen); Subdosierte Antikörper gegen den löslichen T-Zell-Rezeptor (subdosierte Anti-CD2/T11/Leu5-AKs), welcher als Suppressorfaktor beschrieben worden ist, und/oder gegen das lösliche MHC II-Molekül, welches immunsuppressiv ist (subdosierte Anti-HLA-DR/Ia1.12-AKs).

Um die frühzeitige anti-idiotypische Gegenregulation zu verhindern, wird empfohlen, mit dem Konjugat aus Idiotyp bzw. seiner Fab/F(ab')₂-Untereinheit plus D-GL bzw. IgG zu sensibilisieren.

Die frühzeitige Ausschaltung gegensteuernder anti-idiotipischer Antwort kann aber auch durch Impfung mit Anti- Idiotypen, besser jedoch mit deren Fab(F(ab')₂-Untereinheiten, erzielt werden. Diese sollen womöglich mit starkem Adjuvans und i.d. oder s.c. bzw. i.m. injiziert werden. Die Effizienz so modifizierter Vakzine kann durch Adjuvans (Al-Hydroxid, Al-Sesquioxid, Al-Phosphat) noch gesteigert werden.

Vorteilhaft ist noch der Zusatz von hochmolekularem PEG und/oder PVP direkt oder in Kombination mit Adjuvans (Adsorbat-Vakzinen), da diese Polymere den Kontakt zwischen immunkompetenten Zellen erleichtern und zur Aktivierung von Effektorzellen (Makrophagen, NK-Zellen) führen.

Das erfindungsgemäße Arzneimittel ist ebenso zur Behandlung von viralen Infekten, die von Immunsuppression (Hypo- oder Anergie) begleitet sind, geeignet. Bekanntlich sind Infektionen vielfach von Immunsuppressionen begleitet. Dies gilt in besonders hohem Maße für die retroviralen Erkrankungen. Eine von denen, die AIDS/ARC/LAS-Erkrankungen, wurde besonders gründlich auch hinsichtlich dieser Immundepression untersucht. So steigt die Zahl der CD8-positiven Suppressorzellen bei ARC-Patienten stark an; ein stark erhöhter Leu 2⁺7⁺/Leu 3⁺-Quotient leitet ein Rezidiv ein.

Wie stark die virusbedingte Immunsuppression ist, kann an der hypo- bis anergen Reaktion auf Lebend- und Totvakzinen festgestellt werden.

Die Ausbreitung des Krankheitserregers im Organismus wird durch die immunsuppressive Basiskonstellation des Patienten stark gefördert. Auch die Medikamente bekommen in einem immunsupprimierten Organismus zu wenig Unterstützung seitens des Immunapparates.

Wegen des gravierenden Einflusses der immunsuppressiven Lage auf den Krankheitsverlauf wird daher erfindungsgemäß durch eine Eliminierung bzw. Coeliminierung der reifen, immunkompetenten Suppressorzellen mit Antikörpern und/oder deren Konjugaten (Phase I) und durch eine indirekte in vitro Präaktivierung bzw. eine direkte in vivo Reaktivierung der Patienten-Effektorzellen (Phase II) der krankheitfördernde immunsuppressive Zustand des Patienten beendet. Für die Behandlung sind die oben beschriebenen Komponenten A und B geeignet, wobei in Phase II bzw. als Komponente B jeweils statt Tumorantigen oder HIV das Erreger-Antigen eingesetzt wird.

Zur Therapie von immunkomplex-assoziierten Krankheiten (Autoimmunerkrankungen, Glomerulonephritiden, chronischen Infekten, neoplastischen Erkrankungen) kann die mit oralen Enzympräparaten durchgeführte Therapie durch die in vivo Eliminierung/Coeliminierung von B-Zellen unterstützt bzw. ersetzt werden. Dazu geeignet sind (a)
Anti-CD19- und/oder Anti-CD20- und/oder Anti-CD21- und/oder Anti-CD22-AK bzw. deren Konjugate mit Cytotoxinen ("Immuntoxine").
(b) Kombination von (a) mit Anti-CD4/Leu3/T4-AK bzw. deren Konjugaten mit Cytotoxinen.
(c) Falls das Antigen bekannt ist, wie z.B. bei der gegen die glomeruläre Basalmembran gerichteten Glomerulonephitis, wird das Konjugat aus diesem Antigen bzw. seinem Anti-Idiotypen mit Cytotoxinen und/oder mit starken Tolerogenen (D-GL) verwendet.
(d) Die unter Punkt (a) und (b) aufgeführten Antikörper bzw. Immuntoxine können mit Antikörpern gegen Complement bzw. seine Untereinheiten kombiniert werden.
(e) Mit Fab(F(ab')₂-Untereinheiten (a) der gegen Fc-Rezeptoren (den proteinase-sensitiven und/oder den proteinase-resistenten Rezeptoren) und/oder (b) der gegen Complement-Rezeptor 1 (C3b-spezifisch), 2 (C3d-spezifisch) und 3 (C5a-spezifisch) gerichteten Antikörpern kann die Chemotaxis und Aktivierung von gewebezerstörenden Makrophagen und (neutrophilen) Granulozyten weitgehend verhindert werden. Beispiel eines solchen Fc-Rezeptor erkennenden Antikörpers ist der CD16/Leu11a/b/c-Mab.
(f) Mit der Fc-Untereinheit von IgG(=Fc-gamma) kann die Bindung und Aktivierung von Makrophagen und Granulozyten gehemmt werden.
(g) Mit Anti-alpha2-Macroglobulin und/oder Anti-alpha1-Proteinase-Inhibitor kann die vorzeitige Inaktivierung von Gewebe zerstörenden Proteasen erzielt werden.

Bei entzündlichen Vorgängen im Gelenk, wie sie bei entzündlichen Rheumaerkrankungen (rheumatoide Arthritis) auftreten, spielen Interleucine (IL-1 und IL-2) sowie Leukotriene (z.B. LTB4) eine wichtige Rolle. IL-1 trägt zur Ausscheidung von PGE2 und von Kollagenase bei. LTB4 wirkt chemofaktisch auf Makrophagen. Der von Makrophagen ausgeschiedene PAF ("platelet activating factor") ist ebenso chemofaktisch. Zur Behandlung dieser Erkrankungen eignen sich daher Anti-PAF-Antikörper, Anti-PGE2-Antikörper, Anti-LBT4-Antikörper, Anti-IL-1-Antikörper, Anti-IL-2-Antikörper, Collagen/Tropocollagen-Konjugate mit Cytotoxinen und/oder Tolerogenen (D-GL), alpha2-Macroglobulin und/oder alpha1-Protease-Inhibitor, Anti-CD19 (CD20, CD21, CD22)-AK, allein oder kombiniert mit Anti-CD4/Leu3/T4-AK, Fc-Untereinheit von IgG oder Anti-Complement bzw. Anti-Complement-Untereinheiten.

Das System Idiotyp: Anti-Idiotyp funktioniert schneller und sensibler als das System Antigen: Antigen-erkennende Immunozyten. Die Gegensteuerung der Idiotypen durch Anti-Idiotypen erfolgt durch CTL- bzw. B-artige Mechanismen und nicht durch Bildung von (Anti-Idiotyp)-Suppressorzellen. Anti-Idiotyp entspricht einem hochkonzentrierten Antigen, eher einem bereits prozessierten Antigen (Epitop) auf APC (Makrophagen)-Oberfläche. Bekanntlich ist dieses hochkonzentriert und hoch effizient gegenüber dem nicht-prozessierten, freien Antigen. Durch spezielle Konjugate werden immunrelevante Strukturen auf Immunozyten (T4- und/oder T8-Zellen) "kurzgeschlossen" und die Zellen dadurch aktiviert, z.T. unabhängig von AOC ("intrazelluläre/intramembranale cross-linkers") oder aber die immunkompetenten Zellen werden durch "interzelluläre cross-linkers" gezielt in engen Kontakt gebracht.

Es werden nun neuartige Vakzinen auf der Basis von Anti-Idiotypen und von speziellen intrazellulären (intramembranalen) und/oder interzellulären cross-linkers (Heterokonjugaten) zur Verfügung gestellt werden. Dazu können Anti-Idiotyp-AK oder seine Fab/F(ab')₂-Untereinheit, frei oder gekoppelt als Hapten an stark immunogene Carriers verwendet werden. Es ist vorteilhaft, nicht nur einen, sondern mehrere Anti-Idiotypen, imitierend ("internal mirror image") mehrere Antigene bzw. Epitope des Krankheitserregers (Bakterien, Viren, incl. HIV, Onkoviren usw.) vorzusehen. Ebenso ist es von Vorteil, den gleichen Anti-Idiotypen, bzw. seine Fab/F(ab')₂- Untereinheit, an mehrere verschiedene Carrier zu koppeln.

Um die gegenregulierenden Anti-Idiotypen im voraus zu verhindern und somit die klonale Expansion von das Pathogen erkennenden, für den späteren Impfschutz wichtigen Idiotypen (CTL- und B-Gedächtniszellen) zu erhöhen, wird die Vorbehandlung mit Konjugaten, bestehend aus Idiotyp-(Fab/F(ab')₂- Untereinheiten des xenogenen, gegen das Pathogen gerichteten Antikörpers mit Cytotoxin empfohlen. Alternativ oder zusätzlich hierzu kann der gleiche Idiotyp bzw. seine Untereinheit an starke Tolerogene (z.B. D-GL) gekoppelt werden. Die Konjugate mit Cytotoxinen führen zur Eliminierung der gegensteuernden anti-idiotypischen T- und B-Subklone, die Konjugate mit Tolerogenen verhindern zusätzlich deren Nachrekrutierung.

Eine zusätzliche, z.T. APC (Makrophagen)-unabhängige Aktivierung von T4- und/oder T8-Zellen kann durch "intramembranale cross-linkers", d.h. Konjugate aus Anti-CD3- bzw. Anti-Ti/WT31- bzw. Anti-CD2-plus Anti-CD4- bzw. Anti-CD8-Mabs (oder deren Fab/F(ab')₂-Untereinheiten) erzielt werden. Vorteilhaft ist die Kombination dieser T4- bzw. T8-aktivierenden Konjugate mit Immunkonjugaten, bestehend aus IL-1 bzw. Tp67 (Fab/F(ab')₂-Untereinheit) mit Anti-CD4- bzw. Anti-CD8-Mab (Fab/F(ab')₂) und/oder aus IL-2 mit Anti-CD4- bzw. Anti-CD8-Mab(Fab/F(ab')₂). Von Bedeutung sind auch ein IL-1:IL-2-bzw. ein Tp67: IL-2-Heterokonjugat, sowie Konjugate aus mitogenen Lektinen (z.B. PHA) bzw. deren Untereinheiten und Anti-CD4- bzw. Anti-CD8-Mab (Fab/F(ab')₂. Eine Antigen(Pathogen)-spezifische, APC (Makrophagen)-unabhängige Aktivierung der T4- und T8-Subklone (und via T4-Subklone der B-Subklone) kann durch spezielle Konjugate, bestehend aus Antigen (Bruchteil) bzw. Anti-Idiotyp (Fab/F(ab')₂) mit Anti-CD4- bzw. Anti-CD8 (Fab/F(ab')₂) erfolgen. Bei dieser antigenspezifischen T- und B-Aktivierung werden demnach mitogene Antikörper (z.B. Anti-CD3-T1-Mab) bzw. mitogene Lektine (z.B. PHA) durch Antigen bzw. sein Anti-Idiotyp ersetzt. Eine weitere Form der beschleunigten T4- und T8-Aktivierung stellen "interzelluläre cross-linkers" dar. Diese Konjugate können antigenspezifisch oder -unspezifisch sein. Die antigen-spezifischen Konjugate bestehen aus Antigen (oder seiner Peptid-Untereinheit) einerseits bzw. aus dem entsprechenden Anti-Idiotyp-Antikörper andererseits, mit Anti-CD11c- bzw. Anti-CD14- bzw. Anti-CD15- bzw. Anti-CD16-Mab (oder deren Untereinheiten).

Die antigen-unspezifischen Konjugate bestehen aus mitogenen Antikörpern (Fab/F(ab')₂) (Beispiele: Anti-CD3-, Anti-Ti/WT31-, Anti-CD2-Mab) bzw. aus mitogenen Lektinen (Beispiel: PHA) oder dessen Untereinheiten (Monomeren) mit Anti-CD11c- bzw. Anti-CD14- bzw. Anti-CD15- bzw. Anti-CD16 (oder deren Untereinheiten).

Diese "interzellulären cross-linkers" sind auch im speziellen Falle der Anti-HIV-Vakzination bevorzugt, wobei die antigen-spezifischen Konjugate hier aus HIV-Antigenen, speziell env-Glykopeptiden (oder deren Peptid-Bruchstücken) bzw. entsprechenden Anti-Idiotypen mit Anti-CD11c bzw. Anti-CD14- bzw. Anti-CD15- bzw. Anti-CD16 (oder deren Untereinheiten) zusammengesetzt sind.

Die klonale Expansion von krankheitsschützenden T(CTL)- und B-Subklonen kann auch durch eine Vorbehandlung (2 bis 7 Tage vor der Impfung) des Impflings mit Alkylantien wie Endoxan, dem Konjugat aus Pathogen bzw. seinen oligopeptidischen Untereinheiten mit (D-GL bzw. PEG bzw. isologem/antologem IgG, dem Komjugat aus dem pathogen-imitierenden Anti-Idiotyp (Fab/F(ab')₂-Untereinheit) mit D-GL bzw. PEG bzw. isologem/antologem IgG, PGE2-Inhibitor (Indomethazin, Aspirin), Cimetidin und/oder Theophyllin (Theobromin) günstig beeinflußt werden. Dies gilt auch für Anti-HIV-Vakzinationen.

Anstelle von vorgefertigten "interzellulären cross-linkers" kann durch eine sukzessive Behandlung mit Biotin-haltigen mitogenen Antikörpern (z.B. Anti-CD3- oder Anti-Ti/WT31-AK) bzw. deren Fab/F(ab')₂-Untereinheiten, und mit Avidin-haltigen Antikörpern (Fab/F(ab')₂) des Anti-CD11c (Anti-CD14-, Anti-CD15-, Anti-CD16-)Typs ebenso eine gezielte Interaktion von immunkompetenten Zellen und somit T-Zell-Aktivierung erzielt werden. Dies gilt auch für den speziellen Fall der Anti-HIV-Vakzination.

Durch Verhinderung von gegenregulierenden antigenspezifischen Antikörpern kann die vorzeitige Hemmung der klonalen Expansion von cytotoxischen T-Zellen verhindert werden. Dazu werden Anti-B-Antikörper (mono- und/oder polyklonale) bzw. entsprechende Immuntoxine verwendet.

Mit Anti-CD4-AK und/oder Anti-Leu18-AK kann die klonale Expansion von cytotoxischen T-Zellen, unter denen sich auch ca. 10 % IL-2-produzierende Klone befinden, intensiviert werden. Hierzu werden Anti-CD4-AK und/oder Anti-Leu18-AK bzw. entsprechende Immuntoxine verwendet.

Mit Cytostatika können in der zweiten Phase der Sensibilisierung (6. bis 12. Tag nach Erstimpfung) die zu diesem Zeitpunkt stark proliferierenden antigen-spezifischen Suppressorzellen eliminiert werden, und zwar mit Cytostatika (Alkylantien), speziell Endoxin und/oder Anti-CD25/Tac bzw. IL-2:Cytotoxin-Konjugaten.

Generell gilt, daß sich die Effizienz der Impfung noch wesentlich steigern läßt wenn der Impfstoff mit Adjuvantien kombiniert ist, wenn man einen technisch aufwendigeren Weg wählt, indem man die Peripherblut-Leukozyten des Impflings zuerst in Makrophagen, d.h. adhärierenden Zellen, und Lymphozyten trennt, Makrophagen mit dem Antigen (Pathogen) inkubiert (24 Stunden) und anschließend wieder mit den Lymphozyten vereint und reinjiziert, und wenn man sogenannte "Schachbrett-"Vakzination (Sedlouk) vornimmt. Dies gilt auch für die AIDS/ARC-Impfung.

Bei den bisher vorgesehenen Anti-AIDS-Impfstoffen wurde nichts gegen die Cogenerierung von Suppressorklonen, die bei einer späteren Infektion die begonnene Immunantwort vorzeitig abbrechen, getan. Bekanntlich ist selbst die Proliferation von Vaccinia-Viren, die als Träger(Vektor)-Viren bei der rekombinierten VacciniaHIV-Impfung vorgesehen sind, mit starker Immunsuppression verbunden. Bei den hier vorgesehenen Vakzinen wird diese Schwäche "konventioneller" Anti-AIDS- Impfstoffe systematisch umgangen. Es werden dazu HIV-Antigene (env-, pol- und/oder gag-Antigene) bzw. die entsprechenden Anti-Idiotypen, vorteilhafterweise jedoch die 6 bis 20 Aminosäuren enthaltenden Peptidbruchstücke dieser viralen Antigene und/oder die Fab/F(ab')₂-Untereinheiten der entsprechenden Anti-Idiotyp-Antikörper als Haptene zusammen mit stark immunogenen Carriers verwendet. Ein Sonderfall ist die prophylaktische Impfung mit gp120 bzw. deren Peptid-Untereinheiten sowie die Impfung mit Anti-CD4-AK bzw. deren Fab/F(ab')₂-Untereinheit. Die bereits bekannte Vakzination mit Anti-CD4-Antikörpern kann wesentlich verbessert werden, wenn anstelle von Anti-CD4-AK entweder seine Fab/ (F(ab')₂-Untereinheit oder vorteilhafterweise ein Konjugat mit einem oder besser mehreren stark immunogenen Carriers und/oder eine Kombination mit anderen, zusätzliche virale Antigene erkennenden Konjugaten vorgesehen wird. Um die gegenregulierenden Anti-Idiotypen im voraus auszuschalten und hierdurch die klonale Expansion von HIV-bekämpfenden cytotoxischen T- und B-Gedächtniszellen zu erhöhen, wird die Vorbehandlung mit Konjugaten aus Idiotypen, d.h. Fab/(F(ab')₂-Untereinheiten der gegen HIV-Strukturen gerichteten Antikörper, plus Cytotoxin empfohlen. Alternativ oder zusätzlich hierzu kann der gleiche Idiotyp bzw. seine Untereinheit an starke Tolerogene (z.B. D-GL) gekoppelt werden. Die Konjugate mit Cytotoxinen führen zur Eliminierung der gegensteuernden anti-idiotypischen T- und B-Subklone, die Konjugate mit Tolerogenen verhindern zusätzlich deren Nachrekrutierung. Eine zusätzliche, zum Teil APC (Makrophagen)-unabhängige Aktivierung von T4- und/oder T8-Zellen kann durch "intramembranale cross- linkers", d.h. Konjugate aus Anti-CD3- bzw. Anti-Ti/WT31- bzw. Anti-CD2- mit Anti-CD4- bzw. Anti-CD8-AKs erzielt werden.

Eine antigen-spezifische T4- bzw. T8-Aktivierung (und via T4 eine B-Aktivierung) kann durch Konjugate aus viralen Antigenen (bzw. deren Peptid- Untereinheiten) und/oder den entsprechenden Anti-Idiotyp-Antikörpern mit Anti-CD4- bzw. Anti-CD8-AK erzielt werden.

Vorteilhaft ist die Kombination dieser T4- bzw. T8-aktivierenden Konjugate mit Immunkonjugaten, bestehend aus IL-1 oder IL-2 bzw. Tp67 (Fab/F(ab')₂-Untereinheit) mit Anti-CD4- oder Anti-CD8-AK (Fab/F(ab')₂). Von Bedeutung sind auch ein IL-1:IL-2- bzw. ein Tp67:IL-2-Heterokonjugat, sowie Konjugate aus mitogenen Lektinen (z.B. PHA) bzw. deren Untereinheiten (Monomeren) mit Anti-CD4- bzw. Anti-CD8-AK (Fab/F(ab')₂). Eine sehr effektive, aber technisch schwerer durchführbare prophylaktische Vakzination sieht vor, aus dem Peripherblut des Impflings zuerst die adhärierenden Zellen (Makrophagen) abzutrennen, sie anschließend in Anwesenheit von inaktivierten freien oder membran-gebundenen HIVs und Makrophagen-Aktiviatoren (z.B. Endotoxin, gamma-Interferon, MDP/MTP-PE) sowie von PGE2(PGE1)-Inhibitoren (Indomethazin, Aspirin) und/oder Anti-PGE2 zu inkubieren und diese HIV-Strukturen präsentierenden Makrophagen direkt oder nach Inaktivierung (Mitomycin C, Bestrahlung, Formaldehyd/Glutaraldehyd) zwecks prophylaktischer Anti-AIDS-Vakzination in den Impfling zu injizieren. Durch eine Vorbehandlung von env-Glykopeptiden, speziell gp120 (gp160) mit Mucopolysaccharidasen (Neuraminidase, Lysozym) kann die Antigenität dieser viralen Strukturen stark erhöht werden, ebenso wie durch direktes Zusetzen von Vakzine.

Bei Knochenmark-Transplantationen, wie sie bei ganzkörperbestrahlten und/oder supraletal chemotherapierten Leukämiepatienten, aber auch bei Patienten mit oplastischer Anämie und jenem mit SCID ("severe combined ummunodeficiency disease") praktiziert werden, müssen die restlichen tumorprotektiven Suppressorzellen analog wie auch sonst bei Tumorpatienten entfernt werden. Auch die in vitro Präaktivierung bzw. in vivo Reaktivierung von immunkompetenten Effektorzellen, die die Proliferation von den residuellen Tumor- und (Prämikro)-metastasenzellen, aber auch die explosionsartige Vermehrung von bakteriellen und viralen Infekten in der kritischen Phase vor der Reetablierung der Patienten-immunabwehr verhindern sollen, zeigt Analogien zur Immunreaktivierung nach Suppressorzell-Depletion, wie oben bei Tumor- und AIDS-Patienten beschrieben.

Durch die unspezifische Prädepletion immunkompetenter Lymphozyten im Rahmen der Ganzkörperbestrahlung bzw. der hochdosierten Chemotherapie kommt man schon mit einem Bruchteil der sonst benötigten Antikörper und Immunstimulantien bzw. regenerierter Effektorzellen aus.

Bei histoinkompatiblen Knochenmark-Spendern kann durch in vitro Prädepletion alloreaktiver T-Zellen, die in einer Art MLC/MLR-Ansatz präaktiviert und anschließend mit Immuntoxinen auf der Basis spezieller, aktivierter T-Zellen erkennender Mab oder des Lymphokins IL-2 lysiert werden. Alternativ hierzu können in vitro allospezifische Suppressorzellen prägeneriert und den Spender-Knochenmarkzellen vor Infusion zugesetzt werden. Diese Prägenerierung der allospezifischen Suppressorzellen erfolgt ebenso in einer Art MLC/MLR-Ansatz, allerdings unter streng immunsuppressiven Bedingungen.

Die zur T-Depletion von restlichen, tumor-spezifischen Suppressor-T-Zellen und Effektorzell-Aktivierung geeigneten Mittel entsprechen jenen bei der Behandlung von Tumor- und AIDS-Patienten beschriebenen.

Für die in vitro Prädepletion von alloreaktiven T-Subklonen sind Anti-CD25/IL-2-Rezeptor-AK, Anti-CD45-AK und/oder Anti-HLA-DR-AK (plus Complement) oder entsprechende Immuntoxine geeignet. Diese Antikörper bzw. Immuntoxine werden 4 bis 6 Tage lang coinkubierten Spender- und Empfänger- Lymphozyten zugesetzt, wobei die alloreaktivierten - und nur diese - T-Zellen in vitro eliminiert werden.

Die allospezifischen Suppressor-T-Zellen können durch eine 6- bis 10tägige Coinkubation von Spender- und Empfänger-Lymphozyten in einem Medium, das Corticosteroide wie Hydrocortison, Prednison, Methylprednisolon, immunsuppressive Cytostatika (Azathioprin), Cyclosporin A, PGE2 (PGE1), Anti-HLA-DR-AK, Anti-CD4-AK, ConA und/oder Anti-IL-2 (Anti-IL-1)enthält prägeneriert und dem Spendermark zugemischt werden.

Die autologe Knochenmark-Transplantation bei soliden Tumoren kann durch CK-2-AK bzw. CK-2-Immuntoxin verbessert werden. Durch Cytokeration-erkennende Antikörper und Immuntoxine können autologe Lymphozyten, aber auch LAK- und TIL- Zellen von kontaminierenden Tumorzellen befreit (purged) werden. Dazu können CK-2-AK und/oder sein Konjugat mit Cytotoxinen eingesetzt werden.

Für alle bisher beschriebenen Verfahren sind noch eine Reihe weitere Konjugate geeignet, die allein oder vorteilhaft als Bestandteil des entsprechenden erfindungsgemäßen Arzneimittels immunstimulierenden und/oder immuntherapeutischen Effekt aufweisen. Sie greifen aktiv in die Immunregulation ein und können eine ganze Reihe von Krankheiten positiv beeinflussen. Einige unter ihnen ermöglichen wesentliche Verbesserungen bei Organ(Nieren)- und/oder Knochenmark-Transplantationen.

Eine Variante sind Immunkonjugate, die aus zielzell-erkennenden Antikörpern und Enzymen, die Cytostatika lokal aktivieren (Zielzellen: Tumorzellen, (retro)viral (z.B. mit HIV) befallene Zellen, Leukozyten-Subpopulationen bestehen. Die Vorstufen bzw. chemisch präinaktivierten Cytostatika ("prodrugs") werden durch Enzyme, die als Konjugate mit Anti-Tumor-AK an die Tumorzielzellen "fixiert" und lokalisiert werden, in situ in ihre aktive, cytotoxische Form umgewandelt (Beispiel: orale, in situ aktivierbare Cytostatika). Das gleiche Prinzip gilt auch für die selektive Eliminierung von Leukozyten-Subklassen (z.B. Suppressor-T-Zellen). In diesem Falle werden die Anti-Tumor-AK durch Anti-Leukozyten-AK im Konjugat ersetzt. Im Falle der selektiven Eliminierung von Leukozyten-Subklassen können die Cytostatika-aktivierenden Enzyme an Cytokine (Monokine, Lymphokine) statt an Antikörper gekoppelt werden.

Eine weitere Variante sind Immunkonjugate, die aus Zielzell-erkennenden Antikörpern und Enzymen, die essentielle Zellmetabolite lokal vor deren Internalisierung abbauen und so die Zielzelle "aushungern" (Zielzellen: Tumorzellen, (retro)viral (z.B. mit HIV) befallene Zellen, Leukozyten-Subpopulationen) bestehen. Diese Immunkonjugat-Klasse kann klassische Immuntoxine ersetzen oder unterstützen; dadurch können etwaige Probleme der RES-Toxizität von Immuntoxinen umgangen werden. Das Prinzip dieser neuartigen Immunkonjugate ist die lokale Verarmung der Zielzellen (Tumorzellen, Leukozyten-Subpopulationen) an essentiellen Zellsubstraten. Beispielsweise geeignet sind Immunkonjugate, die je eines der folgenden Enzyme Asparaginase, Glutaminase, Arginase, Methioninase, beta-Tyrosinease, Phenylalanin-Ammonium-Lyase, Xanthin-Oxidase, Folsäureabbauenden Enzymen (Carboxypeptidase G) und je einen Antikörper, erkennend Membranstrukturen auf den Zielzellen (z.B. Tumorantigene, Differenzierungsantigene usw.) enthalten.

Wenn Leukozyten-Subpopulationen selektiv geschädigt werden sollen, können die zellmetabolite-abbauenden Enzyme an Cytokine (Monokine, Lymphokine) statt an Antikörper gekoppelt werden.

Allgemein ausgedrückt besteht diese Immunkonjugat-Klasse aus Enzymen, die die Zielzelle an metabolischen Präkursoren verarmen läßt und aus entsprechenden AK (bzw. deren Untereinheiten), die diese Enzyme an die Oberfläche der Zielzellen (und nur dieser) binden.

Bei der bekannten "Enzymtherapie", z.B. mit Asparaginase oder Arginase, wird das Enzym als solches injiziert; die Wirkung ist nicht-selektiv und nicht-lokalisiert, die Mengen des benötigten Enzyms entsprechend hoch. Die Wirkung der Enzym-Konjugate mit Zielzell-erkennenden AK bzw. deren Fab/F(ab')₂-Untereinheiten ist lokalisiert auf die Zielzellen und hierdurch selektiv.

In einer weiteren Ausführungsform werden Immunkonjugate, verwendet, die aus Zielzell-erkennenden Antikörpern und Enzymen, die die Zielzellen, insbesondere Leukozyten-Subpopulationen direkt aktivieren. Das Grundprinzip dieser Immunkonjugat-Klasse ist die gezielte "Fixierung" von zielzell-aktivierenden Enzymen an der Oberfläche dieser Zielzellen. Detaillierte Versuche haben gezeigt, daS Hydrolasen (Proteinasen, Lipasen, Mucopolysaccharidasen) Zellen kurzfristig aktivieren können und daß dieses Prinzip von immunkompetenten Zellen selbst zur Steigerung der Aktivität genutzt wird. Der Vorteil dieser und anderer Enzym-haltiger Immunkonjugate ist deren Wirkung nicht nur auf die einzelne, Enzym-beladene Zielzelle, sondern auch auf die unmittelbaren Nachbarzielzellen, die evtl. vom Immunkonjugat nicht "erreicht" worden sind. Aus gleichem Grund sind Radionuklid-haltige Immunkonjugate erfolgreicher als Cytotoxin (Ricin oder Doxorubicin)-haltige.

Hierzu sind Immunkonjugate, bestehend aus je einem hydrolytischen Enzym (Proteinasen, wie Trypsin, Chymotrypsin, Papain, Bromelin; Lipasen oder Mucopolysaccharidasen, wie Sialidase/Neuraminidase/RDE oder Lysozym) und aus je einem zielzell-erkennenden Antikörper geeignet. An die Stelle der Antikörper können Cytokine/Monokine, Lymphokine) treten, da deren Affinität zum Rezeptor noch höher als jene des Antikörpers zum Membranantigen ist.

Um Leukozyten-Subpopulationen als Zielzellen zu aktivieren, können Konjugate, bestehend aus zielzell-erkennenden Antikörpern und zielzell-aktivierenden Komponenten eingesetzt werden, wie z.B. Immunkonjugate, bestehend aus Makrophagen/Monozyten-,NK-Zellen- oder T-Zellen-aktivierenden Immunstimulatoren/Immunpotentiatoren, und je einem zielzell-erkennenden Antikörper, insbesondere Immunkonjugate, bestehend aus Interferon-gamma, Lysolezithin, Endotoxin oder MDP (MTP-PE) und Anti-M1(M3, M5, CR1)-AK oder aus Statolon oder Phorbolester und Anti-CD2-(CD5)-AK.

Mit dieser Immunkonjugat-Klasse werden zellaktivierende Substanzen direkt an die Zielzellen gebracht, wodurch ihre Dosierung relativ niedrig gehalten werden kann.

Weiterhin können für Tumorzellen oder (retro)viral (z.B. mit HIV) befallene Zielzellen Konjugate, die aus zielzell-erkennenden Antikörpern und cytolytischen Cytokinen z.B. dem Monokin TNF oder dem Lymphokin Lymphotoxin bestehen, insbesondere Konjugate von TNF oder Lymphotoxin mit Anti-Tumor-Antikörpern oder von TNF mit Anti-gp120-Antikörpern verwendet werden. Durch diese Art von Konjugaten können cytotoyische (tumorizide) Effektorsubstanzen gezielt an die Zielzelle gebracht werden; die Nebenwirkungen können durch Dosisreduktion stark vermindert werden.

Zur Antigen-spezifischen T-Aktivierung eignen sich Mischungen, die mitogene, die T4:APC-Interaktion sterisch nicht hindernde Antikörper (z.B. Anti-CD5/T1- oder Anti-CD2/ T11-Ak) und/oder Tp67 bzw. IL-1 enthalten. Durch diese Antikörper oder besser deren Fab/F(ab')₂-Untereinheiten können T-Zellen in den "Bereitschaftszustand" gebracht werden und durch hinzukommendes Antigen in selektiver Weise aktiviert werden.

Von besonderer Bedeutung sind noch Konjugate aus jeweiligen Effektorzell-spezifischen Antikörpern und den für die einzelnen Effektorzellen(Makrophagen/Monozyten, NK- und CTL-Zellen) spezifischen Immunstimulatoren (Immunpotentiatoren, BRM ("biological response modifiers"). Als Beispiel seien genannt die Konjugate aus Anti-CD11c/CD14/CD15/-Antikörpern und MDP (oder MTP-PE oder Thioglykoll), die eine selektive Makrophagenaktivierung ermöglichen, und zwar bei Immunstimulator-Dosierungen, die weit unter jener bei der systemischen Gabe liegen und somit die toxischen Nebenwirkungen wesentlich reduzieren. Ein interessantes Beispiel sind auch Konjugate aus Anti-T-Zell-Antikörpern und 2-Mercaptoethanol, da das 2-Mercaptoethanol bei der (polyklonalen) T-Zell-Aktivierung die Makrophagen(faktoren) partiell ersetzen kann. Die Immunstimulator-Applikation in Form von (Hetero)konjugaten hat auch wesentliche Vorteile gegenüber der Enkapsulierung von Liposomen, da Liposomen-Enkapsulate nach der Phagozytose von Liposomen durch Makrophagen den lysosomalen Enzymen ausgesetzt sind und hierbei inaktiviert oder abgebaut werden können. Zudem sind Heterokonjugate im Körper "beweglicher" als Liposomen, die die Blutbahn nicht verlassen können.

Beispiele von Immunstimulatoren sind Tilorone, Propan-Diamin, Mercaptoalkylamine, BL-20803, MA-56, AET, U-25/166, PMA (Phorbol-Myristat-Acetat), Cimetidin, Quinacrin, bakterielle Extrakte, Lipopolysaccharide, Lipid A, Endotoxin, Toxoide, pflanzliche Extrakte (Lentinan, Pachymaran, Carboxymethylpachymaran), KLH, Laetile, Levamisol, Zymosan, TNP-KLH, Statolon, Pyran-Copolymere (Maleinsäure-Divinylether-Copolymere), COAM, Levan, Oxazolon, Hiu I und Wax C.

Von besonderer Bedeutung sind noch Konjugate, bestehend aus Antikörpern gegen die jeweiligen Zielzellen (Makrophagen, T-Zellen, B-Zellen, NK-Zellen) und den spezifischen Cytokinen (Monokinen, Lymphokinen). So sind z.B. Anti-CD11c/CD14/CD15-AKs mit Lymphokinen wie MIF ("migration inhibition factor"), MAF ("macrophage activation factor"), M-CSF ("macrophage colony stimulating factor") und IL-3 sowie gamma-Interferon (s.o.) zu konjugieren, um diese Makrophagen-stimulierenden Cytokine gezielt und subdosiert an die "richtige Adresse", die Targetzelle, zu bringen. Ähnlich sind Anti-B-Zell- AKs mit Lymphokinen wie BCGF ("B cell growth factor") oder BCDF ("B cell differentiation factor") zu konjugieren und zur weiteren Effizienzsteigerung mit Konjugaten, bestehend aus gleichen Antikörpern und IL-1, IL-2 und/oder gamme-IFN, zu kombinieren.

Zur Verbesserung der Therapie mit LAK ("lymphokine-activated killer" cells)/IL-2 (nach S.A. Rosenberg) sind die im folgenden beschriebenen Mittel geeignet. Die von S.A. Rosenberg et al., entwickelte LAK/IL-2- Tumortherapie hat den Nachteil, daß bei der in vitro Generierung von LAK-Zellen die Suppressorzellen mitaktiviert und klonal expandiert werden und daß die LAK-Zellen dem Patienten infundiert werden, dessen Suppressorzellen nicht zuvor eliminiert worden sind, wodurch die Anzahl benötigter LAK-Zellen enorm hoch gehalten werden muß, um den massiven Widerstand der zurückgebliebenen Suppressorzellen zu überwinden. Die späteren Rezidive sind unter diesen Umständen nahezu vorprogrammiert.

Um diese Situation zu verbessern, werden nun erfindungsgemäß bei der in vitro Generierung von LAK-Zellen zur Verhinderung der Cogenerierung von Suppressorzellen Anti-Leu18-AK bzw. ein Gemisch aus 1/2 cytolytischer Dosis aus Anti-Leu8- und 1/2 cytolytischer Dosis aus Anti-Leu18-AK oder aber ein Gemisch aus je 1/3 cytolytischer Dosis aus Anti-Leu3-, Anti-Leu8- und Anti-Leu18-AK sowie IL-2, Anti-PGE2 (Anti-PGE1) und/oder PG-Inhibitoren (Indomethazin, Aspirin) verwendet. Effizienter als Antikörper, die mit Complement gegeben werden müssen, sind entsprechende Immuntoxine.

Da die LAK-Zellen zu 90 % aus NK-Zellen bestehen, kann zur Vermeidung der Cogenerierung von Suppressor-T-Zellen die CD3-positive T-Zell-Fraktion mit mono- und/oder polyklonalen panT-spezifischen Antikörpern (z.B. Anti-CD3-AK, ATG) depletiert werden, oder aber die CD4-positiven bzw. die CD8- positiven Subklone eliminiert werden. Auch hier sind entsprechende Immuntoxine effizienter als die Antikörper, die mit Complement kombiniert werden müssen.

#Die Inaktivierung der radiosensitiven, leu3⁺8⁺18⁺-Inducer-Suppressorzellen durch Vorbestrahlung des in vitro Ansatzes kann die Effizienz der Suppressor-Eliminierung noch weiterhin steigern, ebenso sie der Zusatz von lysosomotropen Substanzen, wie z.B. Monensin, Chloroquin und/oder NH₄Cl. Evtl. kontaminierende Tumorzellen können durch CK-2-AK (bzw. Immuntoxin) eliminiert werden.

Zur Verbesserung der Therapie mit TIL ("tumor-infiltrating lymphocytes")/IL-2 (nach S.A. Rosenberg et al.) können ebenfalls erfindungsgemäße Mischungen eingesetzt werden. Bei dieser Technik ließen sich spätere Rezidive reduzieren, wenn ähnlich wie bei der LAK/IL-2-Technik dafür gesorgt würde, daß in vitro keine Suppressorzell-Subklone cogeneriert würden und in vivo das eingesetzte Cyclophosphamid mit mono- oder polyklonalen Antikörpern bzw. vorzugsweise deren Immunkonjugaten kombiniert würde, um ruhende und Gedächtnis-Suppressorzellen, die für spätere Rezidine verantwortlich sind, mitzueliminieren. Hierzu sind oben zur Verhinderung der Cogenerierung von Suppressorzellen beschriebenen Mischungen und die oben erwähnte Vorbestrahlung der radiosensitiven Inducer- Suppressorzellen geeignet. Zur Miteliminierung von nicht-proliferierenden enddifferenzierten, ruhenden ("resting") und/oder Gedächtnis-Effektor-Suppressorzellen ist eine Mischung aus Cytostatika (z.B. Cyclophosphamid) und mono- oder polyklonalen Antikörpern (besser Immuntoxinen) gegen CD3-positive T-Zellen oder deren CD8-positive Subpopulation geeignet.

Die an die Behandlung von Leukämiepatienten Rezipienten von Knochenmark erinnernde Behandlung von AIDS-Patienten könnte wesentlich verbessert werden, wenn die Ganzkörperbestrahlung mit einer Nachbehandlung mit mono- oder polyklonalen Antikörpern (Immuntoxinen) kombiniert würde und die Patienten mit in vitro prägenerierten bzw. in vivo reaktivierten viruziden Effektorzellen rekonstituiert würden. Hierzu sind die erfindunggemäßen Mittel, die für Tumorpatienten bzw. Organtransplantationen beschrieben wurden, analog geeignet.

Eine Kombination von kutaner Präsensibilisierung und in situ Immunisierung mit dem gemeinsamen Antigen (Hapten), ist speziell geeignet zur Behandlung des Blasencarcinoms. Das Prinzip ist die klonale Präexpansion von antigen- bzw. hapten-spezifischen T-Zellen (CTLs, T_{DTH}/T_{D}), die bei nachfolgender intratumoraler Immunisierung (mit dem gleichen Antigen bzw. Hapten) in erhöhter Zahl und aktiver Form der Immunabwehr zur Verfügung stehen. Als Beispiel kann die TNBS dienen, mit der zuerst eine Präsensibilisierung nach Art des Cutantestes durchgeführt wird und anschließend nach der Blaseninstallierung der gleichen TNBS die prägenerierten T_{DTH}/T_{D}-Zellen zur Blase wandern, um dort die TNB-Haptengruppe-aufweisenden Tumorzellen abzutöten. Hier werden erfindungsgemäß mit Proteinen (z.B. Eutanen Proteinen) und Zellmembranen direkt und schnell reagierende Substanzen, die nach dieser Bindung als stark immunogene Haptene die Entstehung von T_{DTH}/T_{D}-Zellen, d.h. überwiegend CD4-positiven, bei der DTH-Reaktion beteiligten T-Zellen mit cytotoxischen Eigenschaften stark fördern, verwendet. Hierzu gehören alle stark haptenisierenden Verbindungen, u.a. DNCB, TNCB, DNB.B, TNB.B, Picrylchlorid und die am besten geeignete, da wasserlösliche TNBS (Trinitrobenzolsulfonsäure). Ebenfalls geeignet sind Antikörper, Mabs und Mab-Gemische, sowie deren Fab/F(ab')₂-Untereinheiten, die sich an die Zelloberfläche (zuerst Cutanzellen, später Tumorzellen) binden, Lektine und Prolektine bzw. deren Untereinheiten (Monomeren), die sich an Saccharid-Einheiten der Haut und später der Tumorzellen binden sowie nicht-pathogene Viren (z.B. Vaccinia-Virus, NewCastle-Disease-Virus, Influenza-Virus).

Humorale Suppressorfaktoren können einen enormen Einfluß auf die Immunantwort haben und ihre Neutralisierung ist daher wünschenswert.

Die meisten in vitro Tests werden in Abwesenheit des Patientenserums durchgeführt und sind mit den Fehlern bzw. Unsicherheiten eines unbekannten Beitrages von humoralen Suppressorfaktoren des Patientenserums behaftet; die in vitro gemessene Zellaktivität ist meist höher als die tatsächliche in vivo Aktivität. Es wäre daher wichtig, nicht nur die zelluläre Aktivität, sondern auch den Plasmaspiegel von löslichen Suppressorfaktoren zu messen.

Obwohl die Suppressorfaktoren im Einzelnen noch nicht bekannt sind, sind sie grundsätzlich in der löslichen Fraktion der immunrelevanten Membranstrukturen zu erwarten. Hierzu gehören lösliche IL-2-Rezeptoren, lösliche MHC I-Moleküle und lösliche MHC II-Moleküle, lösliche CD4-Moleküle, lösliche CD8-Strukturen, Plasma-Prostaglandine, speziell PGE2 und PGE1, Plasma-Corticosteroide, lösliche CD2-Moleküle, lösliche T9-Substanzen, lösliches beta2-Microglobulin und lösliche Rezeptoren für Cytokine (Monokine und Lymphokine) generell (z.B. lösliche Rezeptoren für IL-1, IL-2, M-CSF, GM-CSF).

Durch Titration dieser löslichen immunsuppressiven Faktoren mit entsprechenden Antikörpern könnte die vom Patientenplasma ausgehende Immunsuppression "neutralisiert" werden. Im Falle der löslichen Cytokine (incl. Interferon) Rezeptoren läßt die Titration mit Cytokinen direkt, nicht mit Antikörpern, Rückschlüsse über die Konzentration der löslichen Cytokin-Rezeptoren im Plasma zu.

Zur Verwendung für diesen Zweck geeignet sind Antikörper gegen lösliche Fraktionen von IL-2-Rezeptor, MHC I, MHC II, CD4, CD8, CD2, T9, beta2-Microglobulin, Prostaglandine und/oder Corticosteroide (Hydrocortison); die Cytokine IL-1, IL-3, (IL-2), M-CSF, GM-CSF sowie Interferon alpha, beta und gamma.

Eine hochspezifische und zugleich immunsuppression-freie Eliminierung der Tumor- bzw. (retro)viral (z.B. HIV-) befallenen Zellen und der sie beschützenden Suppressorzellen ist möglich durch Verwendung von Anti-Tumor (Retrovirus)-Antikörpern bzw. der entsprechenden Immuntoxine (zur Eliminierung von Tumorzellen bzw. von retroviralen Strukturen exprimierender Zielzellen), von Anti-Idiotyp-Antikörpern (gegen Tumorantigene bzw. retrovirale Epitope) oder von Immuntoxinen, bestehend aus Anti-Idiotyp-Fab/F(ab')₂ und Cytotoxin und/oder aus Anti-Idiotyp-Fab/F(ab')₂ und Tolerogen (D-GL) (anstelle von Anti-Idiotypen können entsprechende Antigene bzw. deren Untereinheiten als Bestandteil des Immuntoxins vorgesehen werden). Um eine "Neutralisierung" von Anti-Tumor (Retrovirus)- Antikörper mit den Anti-Idiotypen bzw. deren Immunkonjugaten zu verhindern, müssen die letztgenannten nach mehrtägigem Zeitabstand gegeben werden. Anstelle der Anti-Idiotypen können Lymphozyten, Plasma oder Blut der mit den Leukozyten bzw. Blut des Patienten (A) sensibilisierten Person (B) eingesetzt werden.

Eine schonende, mit mono- und/oder polyklonalen Antikörpern bzw. entsprechenden Immunkonjugaten erzielbare temporäre Immuninkompetenz bringt große Vorteile mit sich. Die T-Zell-Depletion ermöglicht die Beendigung eines pathologischen, immunregulatorisch bedingten Zustandes und eröffnet aufgrund der geringen Resistenz, bedingt durch die Präliminierung immunkompetenter T-Zellen, neuartige und breite Möglichkeiten zur Etablierung krankheitsverhindernder Konstellationen mit vergleichsweise niedrigen Zahlen an in vitro prägenerierten ("maßgeschneiderten") bzw. in vivo reaktivierten Immunozyten.

Zur möglichst effektiven Eliminierung/Coeliminierung der gewünschten funktionellen Subpopulation (z.B. der alloaggressiven T-Zellen im Falle der Organtransplantation bzw. der tumor-spezifischen Suppressorzellen im Falle der neoplastischen Erkrankungen bzw. der autoaggressiven Lymphozyten im Falle der Autoimmunerkrankungen) werden die funktionell bzw. antigen-selektiven Antikörper bzw. Immunkonjugate mit denen kombiniert, die reife T- und/oder B-Zellen generell eliminieren; bei den T-und/oder B-Zellen unspezifisch depletierenden Substanzen kann es sich sowohl um Antikörper (mono- oder polyklonale) als auch um cytotoxische Substanzen wie Cyclophosphamid, handeln. Der Sinn dieser Kombination ist die wesentliche Effizienzsteigerung sowie Resistenzminimierung für die "maßgeschneiderten" Ersatzimmunozyten. Daher wird die Kombination von panT-spezifischen monoklonalen (Anti-CD3-, Anti-CD5-, Anti-CD2-Mabs) bzw. polyklonalen (ATG(ATS, ALG/ALS) Antikörpern mit Anti-Suppressor- bzw. Anti-Idiotyp-Antikörpern bei neoplastischen und retroviralen Erkrankungen) oder - im Falle der Organtransplantationen - mit Anti-CD4- bzw. Anti-CD8-AKs oder aber - im Falle der Antoimmunerkrankungen - mit Anti-CD25/Tac-AKs bzw. IL-2-Cytotoxin-Konjugaten bevorzugt.

Weiterhin können Kombinationspräparate dieser, gegen Suppressor-Vorläuferzellen gerichteten AK mit den oben erwähnten suppressorzellschwächenden Substanzen, wie Indomethazin, Cimetidin u.a. zur "Stabilisierung" der Übergangsphase zwischen Phase I und Phase II eingesetzt werden, obwohl diese "Stabilisierung" normalerweise nicht notwendig ist.

In einer alternativen Ausführungsform werden statt immunsuppressiver Cytostatika nicht-suppressive Anti-Tumor-bzw. HIV-Antikörper verwendet. Dadurch werden tumor- bzw. HIV-protektive Idiotyp-tragende T- und B-Subklone durch Anti-Idiotyp-Antikörper eliminiert bzw. durch deren Untereinheiten inaktiviert. Hierfür können z.B. Anti-Idiotyp-Antikörper (zur Eliminierung von Idiotyp-tragenden T- und B-Zellen) ggf. in Kombination mit Anti-Tumor bzw.-HIV-Antikörper verwendet werden. Zwischen Anti-Idiotyp-Behandlung und Anti-Tumor-AK-Behandlung sollte allerdings ein Zeitintervall von 10 bis 30 Tagen liegen.

Bei der Bekämpfung von neoplastischen, (retro)-viralen und autoimmunen Krankheiten kann als weitere Maßnahme eine Eliminierung der B-Zellen durchgeführt werden. Anders als bei normalen bakteriellen und viralen Infekten ist die Rolle der spezifischen Antikörper bei retroviralen Infekten incl. HIV, persistierenden/chronischen Infekten generell, bei Tumoren sowie bei Antoimmunkrankheiten problematisch. Die Antikörper führen bei persistierenden Krankheiten, d.h. bei solchen Krankheiten, bei denen der Immunapparat mit dem Krankheitserreger bzw. maligne entarteten Zellen nicht fertig wird, zur spezifischen und generalisierten Immunsuppression, inden sie relevante Antigene an der Zelloberfläche markieren und als Immunglobuline und speziell als Immunkomplexe nicht nur die TG-Suppressorzellen spezifisch aktivieren, sondern auch andere Effektorzellen, z.B. die unspezifischtumoriziden NK-Zellen und Makrophagen supprimieren.

Deshalb muß mindestens in der kritischen Phase nach Entfernung der Tumormasse bzw. nach Ganzkörperbestrahlung der Knochenmark-Empfänger, aber auch nach Entfernung der (potentiellen) HIV-Wirtszellen plus HIV-schonenden Suppressorzellen auf Antikörper und somit auf die B-Zellen verzichtet werden.

Eine antigen-spezifische oder generelle B-Zell-Depletion kann jedoch schon als alleinige oder kombinierte Maßnahme bei immunsupprimierten Patienten, d.h. Patienten mit neoplastischen und (retro)-viralen Erkrankungen incl. AIDS/ARC/LAS, eine humorale und mittelbar zelluläre Immun-Depression einleiten.

Zur unspezifischen B-Zell-Eliminierung sind Anti-CD19-, Anti-CD20-, Anti-CD21- und/oder Anti-CD22-AK bzw. die entsprechenden Immunotoxine, allein oder kombiniert mit Anti-CD4/Leu3/ T4-Mab, Anti-PG2, Anti-Arachidonsäure, PG-Inhibitoren (Indomethazin) und/oder Platelet factor 4 geeignet.

Für die tumor-spezifische B-Zell-Eliminierung/Coeliminierung kommen Konjugate aus Tumorantigen bzw. aus entsprechenden Anti-Idiotypen mit Cytotoxin, allein oder kombiniert mit Konjugaten aus gleichem Tumorantigen bzw. Anti-Idiotypen (bzw. Untereinheiten) mit starken Tolerogenen (z.B. D-GL) inbetracht.

Weiterhin wird bei neoplastischen, (retro)-viralen (incl. HIV) Erkrankungen und Autoimmunkrankheiten eine Eliminierung der T-Zellen in betracht gezogen. Ähnlich wie bei B-Zellen ist die Rolle der T-Zellen bei persistierenden Krankheiten (retroviralen, chronischen und neoplastischen sowie autoimmunen Erkrankungen) problematisch, mehr noch, negativ, da die Suppressor-T-Zellen und deren Ausscheidungsprodukte, die humoralen Suppressorfaktoren, eine spezifische und - bei fortgeschrittener Krankheit - eine generalisierte Immunsuppression induzieren und aufrechterhalten. Die humorale Suppression baut dabei auf löslichen Suppressorfaktoren und den bei B-Zellen erwähnten Immunkomplexen (speziell der IgM-Subklasse) auf. Deshalb muß nach der Entfernung der Tumormasse bzw. nach Ganzkörperbestrahlung bei Knochenmark-Empfängern (Leukämiepatienten) an eine "Fernhaltung" der T-Zellen generell gedacht werden.

Wie bei B-Zellen könnte eine T-Zell-Entfernung als solche, allein oder kombiniert mit einer B-Zell-Depletion, eine wesentliche Erleichterung, d.h. Deblockierung des immunsupprimierten Zustandes des Patienten einleiten.

Geeignet hierzu sind Anti-CD3/Leu4/T3-, Anti-CD5/Leu1/T1- und/oder Anti-CD2/Leu5/T11-Mab bzw. entsprechende Immuntoxine und/oder Anti-Thymozyten-Serum (ATS) bzw. Anti-Thymozyten- Globuline (ATG) ggf. in Kombination mit Anti-CD19 (CD20, CD21, CD22)-AK.

Von Tierversuchen her ist bekannt, daß virale Infekte (LCM-Virus) sowohl nach Eliminierung von T4 (L3T4)- als auch nach Depletierung von T8(Lyt2)-T-Zellen vom Immunapparat erfolgreich bekämpft werden. Eine Entkoppelung der T4- und T8-Zellen kann sich sogar positiv auf die Immunabwehr auswirken. Dieses Prinzip eignet sich auch für neoplastische Erkrankungen. Dazu werden Anti-CD4/Leu3/T4-AK oder Anti-CD8/Leu2/T8(T5)-AK, allein oder kombiniert mit Anti-CD19 (CD20, CD21, CD22)-AK verwendet. Weiterhin kommt zur Behandlung von neoplastischen, (retro)-viralen (incl. HIV) Erkrankungen und Autoimmunkrankheiten die Blockierung der zellulären CD4-positiven Suppressor-Inducer-Zellen und der humoralen T4-abhängigen B-Zellen Immunsuppression durch Ausschaltung der Helfer/Inducer (T4)-Zellen in Frage. Durch Ausschaltung (Depletierung) der T4-Zellen kann sowohl die zelluläre als auch die humorale Antwort auf Tumorantigene blockiert werden, was angesichts der von T- und B-Zellen ausgehenden, schwer kontrollierbaren immunsuppressiven Impulse bei Patienten mit neoplastischen und retroviralen Erkrankungen notgedrungen wünschenswert ist. Die tumorizide bzw. viruzide Aktivität muß von unspezifischen Effektorzellen (NK-Zellen, Makrophagen) getragen werden, was durch in vitro präaktivierte bzw. in vivo präaktivierte Effektorzellen, z.B. LAK-Zellen, speziell nach der Entfernung der Tumormasse bzw. nach der Ganzkörperbestrahlung und/oder hochdosierten Chemotherapie (bei Leukämie-Patienten) erfolgreich bewerkstelligt werden kann. Hierzu können Anti-CD4/Leu3/T4-AK, allein oder kombiniert mit Anti-CD19 (CD20, CD21, CD22)-AK bzw. deren Immunkonjugate mit Cytotoxinen verwendet werden.

Zur Deblockierung des supprimierten Immunsystems werden CD4-positiven Helfer/Inducer vom CD8-positiven Suppressor/cytotoxischen T-Zell-Subset entkoppelt, mit oder ohne gleichzeitige Ausschaltung der humoralen Immunreaktion (B-Zell-Depletierung).

Ein zu schnelles und unausgewogenes Zellwachstum kann zum Zelltod führen, ein Prinzip, das z.B. bei Agent Orange praktisch angewandt wurde. Daher kann die Verwendung von Immunkonjugaten aus Anti-Tumor-AKs mit Substanzen wie Auxinen, Gibberelinen, Hydroxynaphthylaminen, aber auch Wachstumshormonen zur Eliminierung von Zellen angewandt werden. Lokal können derartige Wirksubstanzen auch in freier Form appliziert werden. Durch Konjugatbildung können deren Dosierung und toxische Nebenwirkungen essentiell reduziert werden.

Die Wirksamkeit von Cytostatika kann durch Kombination mit mitose-fördernden Faktoren (Umwandlung der Go- ind G₁-Phase) erhöht werden. Ähnliche Substanzen bzw. Konjugate, die ein zum Selbsttod führendes, unausgewogenes Zellwachstum induzieren, können den cytostatika-resistenten Anteil an ruhenden (Go)-Tumorzellen verringern. Daher kann auch die Verwendung von Immunkonjugaten aus Substanzen wie Auxinen, Gibberelinen, Phytohormonen, aber auch Wachstumshormonen und Anti-Tumor-Antikörpern oder die lokale bzw. subdosierte Anwendung der Wirksubstanzen als solche, gefolgt von Chemo(radio)therapie wesentliche therapeutische Fortschritte bringen.

Suppressorzellen lassen sich durch Histamin selektiv aktivieren; durch Anti-Histaminika und/oder AKs, gerichtet gegen den Histamin-Rezeptor läßt sich die Histamin-induzierte Aktivierung verhindern. Zur Hemmung von Suppressorzellen können daher auch Anti-Histaminika und/oder Anti-Histaminrezeptor-Antikörper eingesetzt werden.

Suppressor-T-Zellen haben Rezeptoren für PNA, weshalb sie für PNA-Cytotoxin-Konjugate verwundbar sind und durch Anwendung von Immuntoxinen, bestehend aus PNA und Cytotoxin, eliminiert werden können.

Eine Aktivierung präexistierender Suppressorzellen mit IL-2 und anschließende Eliminierung mit Cytostatika (Endoxan) und/oder Anti-IL-2-Rezeptor-Mab bzw. IL-2-Cytotoxin-Konjugat und Postexpansion von Nicht-Suppressorzellen mit IL-2 ist ebenfalls möglich. Hierfür können präexistierender Suppressorzellen mit IL-2 allein oder kombiniert mit ConA und/oder PNA aktiviert werden; präaktivierte Suppressorzellen mit Anti-CD25- bzw. Anti-T9- bzw. Anti-Leu17-AK und/oder IL-2-Cytotoxin- bzw. Transferrin-Cytotoxin-Konjugat und/oder Cytostatika (Endoxan) eliminiert und die Nicht-Suppressorzellen mit IL-2 und/oder verschiedenen Immunstimulantien und/oder Mitogenen postaktiviert werden.

Aktivierte T-Zellen können neben Anti-CD25-, Anti-Transferrin-Rezeptor- und Anti-HLA-DR-AKs bzw. Immuntoxinen durch Anti-Leu17-AK bzw. seinem Immuntoxin eliminiert werden.

Für Knochenmark-Transplantationen ist eine weitere Variante vorgesehen, bei der eine in vitro Vortrennung der Makrophagen/Monozyten anhand der Adhäsion, und deren Zusatz zum T- und/oder B-depletierten Spendermark erfolgt. In diesem speziellen Falle der Knochenmark-Anreicherung mit immunkompetenten Zellen sind Makrophagen/Monozyten und NK-Zellen die einzigen reifen/immunkompetenten Zellen. Dies verhindert in hohem Maße die Toleranz-(re)induktion. Die Makrophagen und/oder NK-Zellen können präaktiviert sein.

Bei Autoimmunerkrankungen kann Person B mit (a) Blut, (b) Lymphozyten bzw. (c) Plasma des Patienten A sensibilisiert werden und (a) das Blut (b) Lymphozyten bzw. (c) Plasma der Person B dem Patienten A reinjiziert werden. Dadurch werden die klonal präexpandierten autoaggressiven Klone (T- und B-Zellen) eliminiert.

Beim Blasencarcinom können allogene oder xenogene Erythrozyten mit stark-immunogenen Haptenen (z.B. TNBS) vorbehandelt und (a) zur Sensibilisierung (nach Art des Cutantests) und (b) zur später erfolgenden Blaseninstillation verwendet werden. Mit Enzymimmunstimulation können beim Blasencarcinom die tumor-lokalisierten Effektorzellen (Makrophagen, NK-Zellen, TILs) gegen Tumorzellen aktiviert werden.

## Patentansprüche

1. Arzneimittel, **dadurch gekennzeichnet,** daß es neben üblichen Trägerstoffen, Hilfsmitteln und/oder Zusatzstoffen eine Komponente A, die Suppressor-T-Zellen spezifisch oder unspezifisch eliminiert, ausgewählt aus mit Suppressorzellen, deren Oberflächenantigenen oder aktivierten T-Zellen spezifisch oder unspezifisch reagierenden monoklonalen oder polyklonalen Antikörpern und deren Fragmenten allein oder in Form von Konjugaten mit Toxinen, Lektinen, Cytostatica, Tumor- oder Virus-Antigenen oder entsprechenden Anti-Idiotyp-Antikörpern oder Mischungen davon; und eine Komponente B, die Effektorzellen aktiviert, ausgewählt aus Lymphokinen, Monokinen, Cytokinen, Wachstumshormonen oder Konjugaten davon mit Mitogenen, Enzymen, mit Effektorzellen oder deren Antigenen spezifisch bindefähigen Antikörpern oder deren Fragmenten, enthält.

2. Arzneimittel nach Anspruch 1, **dadurch gekennzeichnet,** daß die Komponente A Anti-CD8-, -CD1-, -CD3-, -CD5-, -CD2- und/oder Anti-panT-Antikörper enthält.

3. Arzneimittel nach einem der Ansprüche 1 bis 2, **dadurch gekennzeichnet,** daß die Komponente A Konjugate von mono- oder polyklonalen Antikörpern, die mit Suppressorzellen reagieren, oder deren Fragmenten mit einer cytotoxischen Substanz, Cytostatika, einem Complementfaktor, humanem präaggregiertem IgG, xenogenen Proteinen und/oder Antihistaminika enthält.

4. Arzneimittel nach einem der vorhergehenden Ansprüche, **dadurch gekennzeichnet,** daß Komponente A Tumorantigen, Virusantigen oder die entsprechenden Anti-Idiotyp-Antikörper enthält.

5. Arzneimittel nach einem der vorhergehenden Ansprüche, **dadurch gekennzeichnet,** daß die Komponente A einen Anti-CD8-Antikörper oder ein Konjugat aus Anti-CD8-Ak und einem Cytotoxin enthält.

6. Arzneimittel nach einem der vorhergehenden Ansprüche, **dadurch gekennzeichnet,** daß Komponente B Interleukin 2 enthält.

7. Arzneimittel nach einem der vorhergehenden Ansprüche, **dadurch gekennzeichnet,** daß Komponente B ein Konjugat von mit Effektorzellen spezifisch bindefähigen Antikörpern oder deren Fragmenten oder mitogenen Lektinen mit einem Lymphokin, einem Enzym, Prostaglandin-Inhibitoren und/oder CAMP-Stimulatoren enthält.

8. Arzneimittel nach einem der vorhergehenden Ansprüche, **dadurch gekennzeichnet,** daß Komponente B Heterokonjugate von Anti-CD3-AK, Anti-Ti-AK oder deren Fragmenten und/oder mitogenen Lektinen mit HLA-DR enthält.

9. Arzneimittel nach einem der vorhergehenden Ansprüche, **dadurch gekennzeichnet,** daß Komponente B Heterokonjugate von Anti-CD8-AK oder mitogenen Lektinen mit HLA-A,B,C enthält.

10. Arzneimittel nach einem der vorhergehenden Ansprüche, **dadurch gekennzeichnet,** daß Komponente B ein Heterokonjugat von Interleukin 2 mit einem Mitogen oder einem Anti-Idiotyp-Antikörper ist.

11. Arzneimittel nach einem der vorhergehenden Ansprüche, **dadurch gekennzeichnet,** daß als Komponente B Heterokonjugate, die T4- bzw. T8-Zellen durch Überbrückung immunrelevanter Membranstrukturen aktivieren, enthält.

12. Verwendung von Suppressorzellen eliminierenden Substanzen, ausgewählt aus mit Suppressorzellen, deren Oberflächenantigenen oder aktivierten T-Zellen spezifisch oder unspezifisch reagierenden monoklonalen oder polyklonalen Antikörpern und deren Fragmenten allein oder in Form von Konjugaten mit Toxinen, Lektinen, Cytostatica, Tumor- oder Virus-Antigenen oder entsprechenden Anti-Idiotyp-Antikörpern oder Mischungen davon; und Effektorzellen aktivierenden Substanzen ausgewählt aus Lymphokinen, Monokinen, Cytokinen, Enzymen, mit Effektorzellen oder deren Antigenen spezifisch bindefähigen Antikörpern oder deren Fragmenten, zur Herstellung eines Arzneimittels gemäß einem der Ansprüche 1 bis 11 zur Behandlung von Krebs, AIDS und viralen Infektionen.

## Claims

1. Drug, **characterized in that** it comprises, in addition to usual carriers, adjuvants and/or additives, a component A eliminating suppressor T-cells in a specific or non-specific way and which is selected from monoclonal or polyclonal antibodies or their fragments reacting in a specific or non-specific way with suppressor cells, their surface antigens, or activated T-cells, or conjugates of said antibodies or their fragments with toxins, lectins, cytostatic agents, tumor or virus antigens or corresponding anti-idiotypic antibodies, or mixtures thereof; and a component B activating effector cells and selected from lymphokines, monokines, cytokines, growth hormones or conjugates thereof with mitogens, enzymes, antibodies, or their fragments, specifically binding to effector cells or their antigens.

2. Drug according to claim 1,
**characterized in that** component A comprises anti-CD8, -CD1, -CD3, -CD5, -CD2 and/or anti-panT antibodies.

3. Drug according to anyone of claims 1 or 2,
**characterized in that** component A comprises conjugates of mono- or polyclonal antibodies, or their fragments, reacting with suppressor cells, with a cytotoxic substance, cytostatic agents, a complement factor, human preaggregated IgG, xenogeneic proteins and/or antihistaminic agents.

4. Drug according to anyone of the preceding claims,
**characterized in that** component A comprises tumor antigen, virus antigen, or the corresponding anti-idiotypic antibodies.

5. Drug according to anyone of the preceding claims,
**characterized in that** compoent A comprises an anti-CD8 antibody or a conjugate of an anti-CD8 antibody with a cytotoxin.

6. Drug according to anyone of the preceding claims,
**characterized in that** component B comprises interleukin 2.

7. Drug according to anyone of the preceding claims,
**characterized in that** component B comprises a conjugate of antibodies, or their fragments, specifically binding to effector cells, or of mitogenic lectins, with a lymphokine, an enzyme, prostaglandin inhibitors and/or cAMP stimulants.

8. Drug according to anyone of the preceding claims,
**characterized in that** component B comprises heteroconjugates of anti-CD3 antibodies, anti-Ti antibodies or their fragments, and/or mitogenic lectins, with HLA-DR.

9. Drug according to anyone of the preceding claims,
**characterized in that** component B comprises heteroconjugates of anti-CD8 antibodies, or mitogenic lectins, with HLA-A,B,C.

10. Drug according to anyone of the preceding claims,
**characterized in that** component B is a heteroconjugate of interleukin 2 with a mitogenic agent or an anti-idiotypic antibody.

11. Drug according to anyone of the preceding claims,
**characterized in that** component B comprises heteroconjugates activating T4- or T8-cells by bridging immunologically relevant membrane structures.

12. Use of suppressor cell eliminating substances which are selected from monoclonal or polyclonal antibodies and their fragments reacting in a specific or non-specific way with suppressor cells, their surface antigens, or activated T-cells either alone or in the form of conjugates with toxins, lectins, cystostatic agents, tumor or virus antigens or corresponding anti-idiotypic antibodies, or mixtures thereof; and from effector cell activating substances selected from lymphokines, monokines, cytokines, enzymes, antibodies or their fragments specifically binding to effector cells or their antigens, in the manufacture of a drug according to anyone of claims 1 to 11 for treating cancer, AIDS, and viral infections.

## Revendications

1. Médicament, **caractérisé par le fait qu**'il contient, outre les porteurs, adjuvants et/ou additifs courants, un composant A qui élimine les cellules T suppressives de manière spécifique ou non-spécifique et qui est choisi parmi les anticorps monoclonaux ou polyclonaux ou leurs fragments réagissant de manière spécifique ou non-spécifique avec des cellules suppressives, leurs antigènes de surface ou des cellules T activées, ces anticorps étant contenus comme tels ou sous forme de conjugués avec des toxines, lectines, cytostatiques, antigènes tumoraux ou viraux ou leurs anticorps anti-idiotypes ou sous forme d'un mélange; et un composant B qui active les cellules effectrices et qui est choisi parmi les lymphokines, monokines, cytokines, hormones de croissance ou des conjugués de ces premiers avec des mitogènes, des enzymes, des anticorps ou leurs fragments capables de se lier spécifiquement aux cellules effectrices ou leurs antigènes.

2. Médicament selon la revendication 1,
**caractérisé par le fait que** le composant A contient des anticorps anti-CD8, -CD1, -CD3, -CD5, -CD2 et/ou anti-panT.

3. Médicament selon l'une des revendications 1 à 2,
**caractérisé par le fait que** le composant A contient des conjugués d'anticorps mono-ou polyclonaux ou leurs fragments réagissant avec des cellules suppressives avec une substance cytotoxique, des cytostatiques, un facteur de complément, une IgG humaine préagrégée, des protéines xénogènes et/ou des antihistaminiques.

4. Médicament selon l'une des revendications précédentes,
**caractérisé par le fait que** le composant A contient un antigène tumoral ou viral ou l'anticorps anti-idiotype correspondant.

5. Médicament selon l'une des revendications précédentes,
**caractérisé par le fait que** le composant A contient un anticorps anti-CD8 ou un conjugué d'un anticorps anti-CD8 avec une cytotoxine.

6. Médicament selon l'une des revendications précédentes,
**caractérisé par le fait que le** composant B contient de l'interleukine 2.

7. Médicament selon l'une des revendications précédentes,
**caractérisé par le fait que** le composant B contient un conjugué d'anticorps ou de leurs fragments capables de se lier spécifiquement à des cellules effectrices ou de lectines mitogéniques avec une lymphokine, une enzyme, un inhibiteur de prostaglandine et/ou des stimulants de cAMP.

8. Médicament selon l'une des revendications précédentes,
**caractérisé par le fait que** le composant B contient des hétéro-conjugués d'anticorps anti-CD3, anti-Ti ou leurs fragments et/ou de lectines mitogéniques avec HLA-DR.

9. Médicament selon l'une des revendications précédentes,
**caractérisé par le fait que** le composant B contient des hétéro-conjugués d'anticorps anti-CD8 ou de lectines mitogéniques avec HLA-A,B,C.

10. Médicament selon l'une des revendications précédentes,
**caractérisé par le fait que** le composant B est un hétéro-conjugué d'interleukine 2 avec un mitogène ou un anticorps anti-idiotype.

11. Médicament selon l'une des revendications précédentes,
**caractérisée par le fait que** le composant B contient des hétéro-conjugués activant les cellules T4 ou T8 par pontage de structures de leurs membranes intervenant dans la réponse immunitaire.

12. Emploi de substances éliminant des cellules suppressives, ces substances étant choisies parmi les anticorps monoclonaux ou polyclonaux ou leurs fragments réagissant de manière spécifique ou non-spécifique avec des cellules suppressives, leurs antigènes de surface ou des cellules T activées, ces anticorps étant choisis comme tels ou sous forme de conjugués avec des toxines, lectines, cytostatiques, antigènes tumoraux ou viraux ou leurs anticorps anti-idiotypes ou sous forme d'un mélange; et parmi des substances activant des cellules effectrices et choisies parmi des lymphokines, monokines, cytokines, enzymes, anticorps ou leurs fragments capables de se lier spécifiquement aux cellules effectrices ou leurs antigènes, en vue de fabriquer un médicament selon l'une des revendications 1 à 11 pour traiter le cancer, le SIDA et des infections virales.
